# EUROPEAN PATENT SPECIFICATION

(11) **EP 2 111 218 B1**
(45) Date of publication and mention of the grant of the patent: **02.07.2014**
(21) Application number: 08700945.2
(22) Date of filing: 24.01.2008
(51) Int. Cl.: C12Q 1/68, C12N 15/88

(54) **DNA CONTROLLED ASSEMBLY OF LIPID MEMBRANES**
DNA-KONTROLLIERTE ANORDNUNG VON LIPIDMEMBRANEN
ASSEMBLAGE DE MEMBRANES LIPIDIQUES RÉGULÉ PAR ADN

(30) Priority: 24.01.2007 DK 200700110
(43) Date of publication of application: 28.10.2009
(73) Proprietor: Syddansk Universitet, 5230 Odense M (DK)
(72) Inventor: VOGEL, Stefan, DK-5000 Odense C (DK); SIMONSEN, Adam Cohen, DK-5000 Odense C (DK)
(74) Representative: Plougmann & Vingtoft A/S
(86) International application number: PCT/DK2008/050011
(87) International publication number: WO 2008/089771

(56) References cited:
- WO-A-01/57064
- WO-A-03/105765
- WO-A-2004/105783
- WO-A-2005/082922
- WO-A-2005/084210
- WO-A-2006/094203
- US-B1- 6 284 267
- US-B1- 6 379 965
- KURZ ANKE ET AL: "Lipid-anchored oligonucleotides for stable double-helix formation in distinct membrane domains." ANGEWANDTE CHEMIE (INTERNATIONAL ED. IN ENGLISH) 3 JUL 2006, vol. 45, no. 27, 3 July 2006 (2006-07-03), pages 4440-4444, XP002459375 ISSN: 1433-7851

## Description

### Field of the invention

The invention relates to oligonucleotides and their use in detection and quantification of nucleic acids.

### Background

The further development of methods for sequence specific detection of nucleic acids is of outermost importance in various fields of life science. In particular such methods are used in relation to genetic diseases and for identification of disease causing pathogens. Also in the further development of personalized medicine, such methods are of relevance.

Very often PCR (polymerase chain reaction) is used for sequence specific detection of nucleic acids. The function of PCR may be to increase the concentration of the target nucleic acid before sequence specific detection of particular genetic markers, and the PCR may itself also be used for detection of particular markers. Detection may also be based on hybridisation of probes that are fluorescently labelled.

Both way, PCR and related detection techniques require sophisticated machinery and often are more time consuming than desirable.

There is clearly a need for improved methods of detecting nucleic acids, and of particular interest are more simple and low cost methods with improved sensitivity.

In addition to being used in relation to detection, oligonucleotides may also be used as therapeutics compounds. Examples are antisense oligonucleotides, siRNAs, microRNAs, ribozymes etc. The compounds all have the promise of being able to sequence specifically target a cellular nucleic acid, typically an mRNA.

A draw back of therapeutic oligonucleotides is their poor delivery into cells, due to their polyanionic nature. Therefore, delivery is often sought mediated by lipids, e.g. in the form of liposomes or by conjugation of a lipid to the oligonucleotide. Still, drawbacks exist. Thus, an oligonucleotide to which a lipid has been conjugated will have non-desirable characteristics because the lipid will give the oligonucleotide a tendency to aggregate, i.e. the lipids of different oligonucleotides will aggregate because of their hydrophobicity.

KURZ et al., "Lipid-anchored oligonucleotides for stable double-helix formation in distinct membrane domains.", (ANGEWANDTE CHEMIE, 3 JUL 2006, vol. 45, no. 27, pages 4440-4444), WO 2005/082922, WO 03/105765, all discloses methods for constructing structures comprising amphiphilic oligonucleotides anchored to lipid membrane, liposomes, vesicles, micelles.

### Summary of the invention

The present invention relates to an amphiphilic oligonucleotide comprising two membrane anchors and methods for using the aforementioned oligonucleotide. In a first aspect, a method for constructing oligonucleotide-lipid membrane architectures is shown. Said oligonucleotide-lipid membrane architectures are useful for detection and quantification of a target nucleic acid. The oligonucleotide-lipid membrane architecture can also be used for separation of a target nucleic acid from a mixture.

### Brief Description of Drawings.

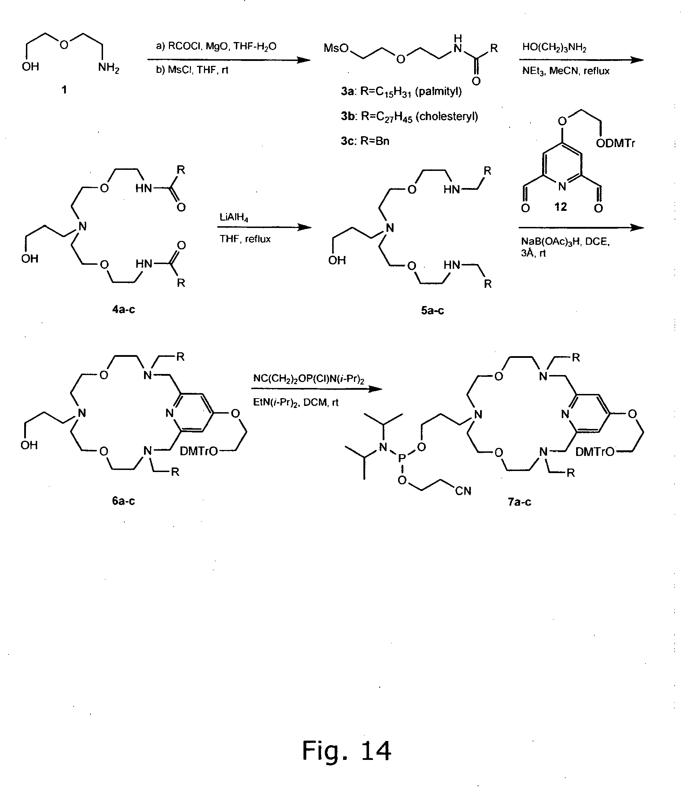

Fig. 1.
   General Chemical core structure of a membrane anchor exemplified as modification X.
Fig. 2.
   Table of exemplified sequences.
Fig. 3.
   Mode of action for the interaction of sequences (ID 3 and ID 4) with lipid bilayers (exemplified using vesicles in the size of 30, 50, 100, 200 and 400 nm).
Fig. 4.
   Mode of action for the interaction of sequences (ID 3 and ID 4) with lipid bilayers (exemplified using vesicles in the size of 30, 50, 100, 200 and 400 nm) when hybridised to a target nucleic acid as exemplified for DNA (sequence ID 1, ID2, ID 5 and ID 6)
Fig. 5.
   Mode of action for the interaction of sequences (ID 3 and ID 4) with lipid bilayers (exemplified using vesicles in the size of 30, 50, 100, 200 and 400 nm) when hybridised to a target nucleic acid as exemplified for DNA (sequence ID 1, ID2, ID 5 and ID 6) and the mode of action for the lipid bilayer assembly process as result of the hybridisation of the probe sequence (sequence ID 3 and ID 4) with any of the exemplified target sequence (sequence ID 1, ID2, ID 5 and ID 6)
Fig. 6.
   Thermal denaturation curves recorded in a UV experiment at 260 nm wavelength showing hybridisation of sequence ID1 and ID2 compared to the hybridisation of sequence ID3 and ID4
Fig. 7.
   First derivative of the thermal denaturation curves recorded in a UV experiment at 260 nm wavelength showing hybridisation of sequence ID1 and ID2 compared to the hybridisation of sequence ID3 and ID4
Fig. 8.
   Thermal denaturation curves recorded in a UV experiment at 260 nm wavelength showing hybridisation of sequence ID1 and ID2 compared to the hybridisation of sequence ID3 and ID4 and run in two consecutive cycles from 15°C to 80°C. showing the reproducibility of the assembly and disassembly process of the bilayers used (vesicles).
Fig. 9.
   First derivative of the thermal denaturation curves recorded in a UV experiment at 260 nm wavelength showing hybridisation of sequence ID1 and ID2 compared to the hybridisation of sequence ID3 and ID4 and run in two consecutive cycles from 15°C to 80°C. showing the reproducibility of the assembly and disassembly process of the bilayers used (vesicles).
Fig. 10.
   Thermal denaturation curves recorded in a UV experiment at 260 nm wavelength showing hybridisation of sequence ID4 and ID1 compared to the hybridisation of sequence ID4 and ID5 at 0,125 µM DNA concentration of each single stranded DNA sequence
Fig. 11.
   Thermal denaturation curves recorded in a UV experiment at 260 nm wavelength showing hybridisation of sequence ID3 and ID2 compared to the hybridisation of sequence ID3 and ID6 at 0,125 µM DNA concentration of each single stranded DNA sequence
Fig. 12.
   Thermal denaturation curves recorded in a UV experiment at 260 nm wavelength showing hybridisation of sequence ID4 and ID1 compared to the hybridisation of sequence ID4 and ID5 at 12,5 nM DNA concentration of each single stranded DNA sequence.
Fig. 13.
   Thermal denaturation curves recorded in a UV experiment at 260 nm wavelength showing hybridisation of sequence ID3 and ID2 compared to the hybridisation of sequence ID3 and ID6 at 0,125 µM DNA concentration of each single stranded DNA sequence
Fig. 14.
   Synthesis of macrocylic building blocks as membrane anchor units in DNA-conjugates (sequence ID 3 and sequence ID 4)
Fig. 15.
   Synthesis of a building block for the synthesis of macrocycles in Figure 14 as membrane anchor units in DNA-conjugates (sequence ID 3 and sequence ID 4)
Fig. 16.
   Thermal denaturation curves for testing target oligonucleotide mediated liposome assembly. See example 8.
Fig. 17.
   Thermal denaturation curves for mismatch discrimination studies. See example 9.
Fig. 18.
   Thermal denaturation curves of an Anthrax sequence. See example 10.
Fig. 19.
   Thermal denaturation curves of Staphlycoccus aureus sequences. See example 11.
Fig. 20.
   Thermal denaturation curves of a Staphlycoccus aureus sequence. See example 12.
Fig. 21.
   3-strand design for liposome assembly. See example 13.
Fig. 22.
   Thermal denaturation curves for 3-strand design. Se example 13.

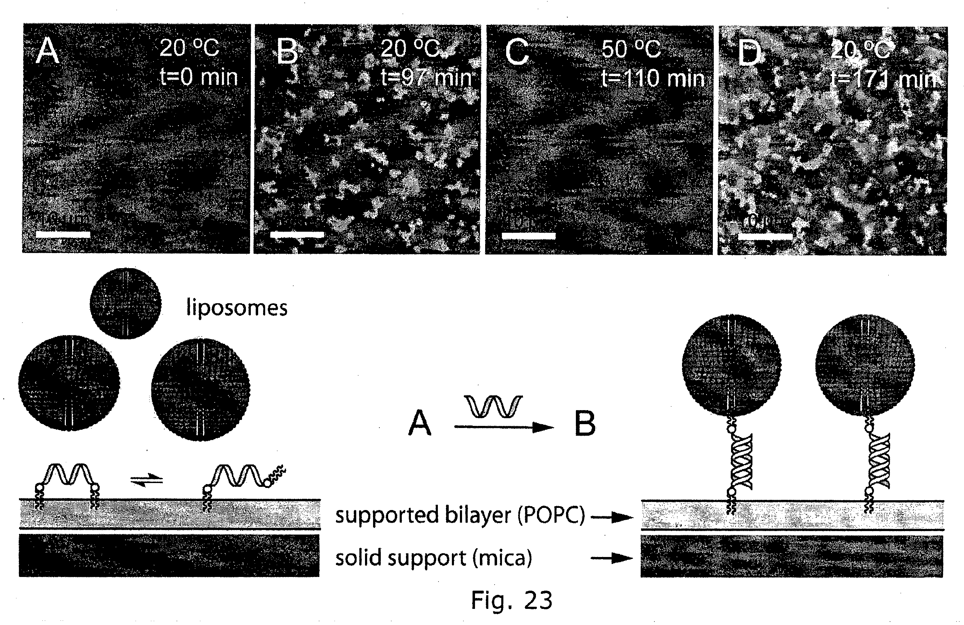

Fig. 23. Immobilisation of oligonucleotides and liposomes on a solid support. See example 14.

### Detailed description of the invention

### Method for constructing oligonucleotide-lipid architectures

In a first aspect, the present disclosure provides a method for constructing oligonucleotide-lipid architectures comprising the steps of:
a) Providing one or more amphiphilic oligonucleotides, together comprising at least two membrane anchors
b) Providing a lipid membrane
c) Incubating the amphiphilic oligonucleotide with the lipid membrane, under conditions allowing insertion of the membrane anchors into the lipid membrane.

When performing steps a-c, the membrane anchors of the one or more amphiphilic oligonucleotides will insert into the lipid membrane. The resulting oligonucleotide-lipid membrane architecture has a number of applications, as will be clear from the embodiments described below.

The method further comprises incubating, under conditions of hybridisation, the one or more amphiphilic oligonucleotides and the lipid membrane with a target nucleic acid that comprises a region of complementarity to the one or more amphiphilic oligonucleotides. The effect of doing so is target nucleic acid controlled lipid membrane aggregation, i.e. the bringing together of lipid membranes. Thus, hybridisation of the amphiphilic oligonucleotide to the target nucleic acid can be detected by detecting lipid membrane aggregation. A major advantage of this mechanism is that lipid membrane aggregation can, at certain concentrations of lipid membranes, amphiphilic oligonucleotides and target nucleic acids, actually be detected by visual inspection of the sample, as the sample will turn milky. Thus, hybridisation and ultimately, the presence of the target nucleic acid can be detected by visual inspection, without sophisticated equipment.

Conditions of hybridisation are well-known to the skilled man. Adjustable parameters include e.g. temperature and ionic strength.

When referring to a "region of complementarity", what is meant is a region that can form a duplex with the amphiphilic oligonucleotide through base pairing. Preferred base pairs are G:C, C:G, A:T, T:A, A:U, U:A, G:U and U:G. In some embodiments, universal bases that allow base pairing to all natural occurring bases are included in either the amphiphilic oligonucleotide or the target nucleic acid.

When more than one amphiphilic oligonucleotide is employed, they are preferably capable of forming a contiguous double stranded complex with the region of complementarity of the target nucleic acid. In some embodiments, a gap of 1, 2, 3 or 4 nucleotides may be present between the amphiphilic oligonucleotides, when base paired to the target nucleic acid. In another embodiment, two amphiphilic oligonucleotides may be separated by one or more oligonucleotides that do not comprise a membrane anchor. The multi-oligonucleotide structures will all prevent the two membrane anchors from inserting into the same lipid membrane, wherefore they will promote lipid assembly.

### The amphiphilic oligonucleotide

In a preferred embodiment, the method only employs one amphiphilic oligonucleotide. As will be apparent, many of the embodiments described below, which is related to only one amphiphilic oligonucleotide will also be useful for embodiments that employs two or more amphiphilic oligonucleotides.

Preferably, the amphiphilic oligonucleotide comprises only 2 membrane anchors.

The two anchors of the amphiphilic oligonucleotide should be separated by at least 5 nucleotides. Otherwise, there may be steric problems in the insertion of both anchors into the lipid membrane. Thus, in a preferred embodiment, two anchors of the amphiphilic oligonucleotide are separated by a distance selected from the group consisting of at least 5 nucleotides, at least 6 nucleotides, at least 7 nucleotides, at least 8 nucleotides, at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, at least 20 nucleotides, at least 21 nucleotides, at least 22 nucleotides, at least 23 nucleotides, at least 24 nucleotides, at least, 25 nucleotides, at least 26 nucleotides, at least 27 nucleotides, at least 28 nucleotides, at least 29 nucleotides and at least 30 nucleotides..

In another preferred embodiment, two anchors of the amphiphilic oligonucleotide are separated by a distance selected from the group consisting of less than 6 nucleotides, less than 7 nucleotides, less than 8 nucleotides, less than 9 nucleotides, less than 10 nucleotides, less than 11 nucleotides, less than 12 nucleotides, less than 13 nucleotides, less than 14 nucleotides, less than 15 nucleotides, less than 16 nucleotides, less than 17 nucleotides, less than 18 nucleotides, less than 19 nucleotides, less than 20 nucleotides, less than 21 ' nucleotides, less than 22 nucleotides, less than 23 nucleotides, less than 24 nucleotides, less than, 25 nucleotides, less than 26 nucleotides, less than 27 nucleotides, less than 28, less than 29 nucleotides, less than 30 nucleotides, less than 40 nucleotides and less than 50 nucleotides.

The amphiphilic oligonucleotide may comprise monomers selected from the group consisting of DNA monomers, RNA monomers, LNA monomers, PNA monomers, INA monomers and morpholino monomers. LNA, PNA, INA and morpholino monomers may be used to increase the melting temperature of the amphiphilic oligonucleotide hybridised to a complementary nucleic acid. Other nucleotide monomers may also be present in the amphiphilic oligonucleotide.

In a preferred embodiment, the length of the amphiphilic oligonucleotide is less than 35 nucleotides. In another embodiment, the length of the amphiphilic oligonucleotide is selected from the group consisting of less than 6 nucleotides, less than 7 nucleotides, less than 8 nucleotides, less than 9 nucleotides, less than 10 nucleotide, less than 11 nucleotides, less than 12 nucleotides, less than 13 nucleotides, less than 14 nucleotides, less than 15 nucleotides, less than 16 nucleotides, less than 17 nucleotides, less than 18 nucleotides, less than 19 nucleotides, less than 20 nucleotides, less than 21 nucleotides, less than 22 nucleotides, less than 23 nucleotides, less than 24 nucleotides, less than, 25 nucleotides, less than 26 nucleotides, less than 27 nucleotides, less than 28, less than 29 nucleotides, less than 30 nucleotides, less than 40 nucleotides and less than 50 nucleotides.

In yet another preferred embodiment, the length of the amphiphilic oligonucleotide is selected from the group consisting of at least 5 nucleotides, at least 6 nucleotides, at least 7 nucleotides, at least 8 nucleotides, at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, at least 20 nucleotides, at least 21 nucleotides, at least 22 nucleotides, at least 23 nucleotides, at least 24 nucleotides, at least, 25 nucleotides, at least 26 nucleotides, at least 27 nucleotides, at least 28 nucleotides, at least 29 nucleotides and at least 30 nucleotides.

The length of the region of complementarity can be adjusted e.g. for optimization of the melting temperature of the amphiphilic oligonucleotide hybridised to the target nucleic acid. Thus, in preferred embodiment, the region of complementarity has a length selected from the group consisting less than 6 nucleotides, less than 7 nucleotides, less than 8 nucleotides, less than 9 nucleotides, less than 10 nucleotides, less than 11 nucleotides, less than 12 nucleotides, less than 13 nucleotides, less than 14 nucleotides, less than 15 nucleotides, less than 16 nucleotides, less than 17 nucleotides, less than 18 nucleotides, less than 19 nucleotides, less than 20 nucleotides, less than 21 nucleotides, less than 22 nucleotides, less than 23 nucleotides, less than 24 nucleotides, less than, 25 nucleotides, less than 26 nucleotides, less than 27 nucleotides, less than 28, less than 29 nucleotides, less than 30 nucleotides, less than 40 nucleotides and less than 50 nucleotides.

In yet another embodiment, the region of complementarity has a length selected from the group consisting of at least 5 nucleotides, at least 6 nucleotides, at least 7 nucleotides, at least 8 nucleotides, at least 9 nucleotides, at least 10 nucleotides, at least 11 nucleotides, at least 12 nucleotides, at least 13 nucleotides, at least 14 nucleotides, at least 15 nucleotides, at least 16 nucleotides, at least 17 nucleotides, at least 18 nucleotides, at least 19 nucleotides, at least 20 nucleotides, at least 21 nucleotides, at least 22 nucleotides, at least 23 nucleotides, at least 24 nucleotides, at least, 25 nucleotides, at least 26 nucleotides, at least 27 nucleotides, at least 28 nucleotides, at least 29 nucleotides and at least 30 nucleotides.

As mentioned above the length of the region of complementarity as well as nucleotide analogues can be used to adjust the melting temperature of the amphiphilic oligonucleotide hybridised to the target nucleic acid.

In a preferred embodiment, the melting temperature is within a range of 5 ° C of the temperature in which the method is performed. Even more preferred is a range of 3 °C or 2°C. By using such a melting temperature, specificity is assured and only perfect hybridisation with no mismatches will occur.

As will be clear from the examples section, the method of the invention gives a very steep (or sharp) melting curve of the amphiphilic oligonucleotide hybridised to a target nucleic acid in the presence of a lipid membrane. Not intended to be bound by theory, it is believed that the steep melting curve is caused by double cooperativity introduced by the presence of the lipid membrane. It is one object of the invention to use this improved discrimination between a perfect duplex and a duplex with one or more mismatches e.g. for more specific detection and quantification of a target nucleic acid.

In a preferred embodiment, the region of complementarity hybridised to a complementary RNA oligonucleotide has a melting temperature of at least 30 ° C, such as at least 40 ° C, such as at least 50 ° C, such as at least 60 ° C.

### Membrane anchors

As described above, the membrane anchors should be capable of insertion into a lipid membrane. Thus, membrane anchors are defined herein as moieties build into an oligonucleotide with the purpose of allowing attachment of the oligonucleotide to a lipid membrane.

In a preferred embodiment, membrane anchors are inserted as monomer building blocks during oligonucleotide synthesis. Thus, membrane anchors may comprise any lipophilic group that will allow insertion into a lipid membrane. Such lipophilic groups are known to the skilled man and may e.g. be fatty acids, steroids etc.

In a preferred embodiment, at least 1 membrane anchor of the amphiphilic oligonucleotide inserts reversibly into the lipid membrane, such that the particular membrane anchor may change position from one lipid membrane to another lipid membrane.

Reversible insertion into a lipid membrane is simple to achieve and demands that the lipohilic group of the membrane anchor does not comprise lipophilic groups of excessive size.

A simple method to test for reversibility is based on aggregation of lipid membranes, as outlined in the examples section. If not at least one membrane anchor inserts reversibly into the lipid membrane, lipid membrane aggregation cannot be achieved, which is easily detected.

In a preferred embodiment, at least one membrane anchor comprise an alkyl chain with a length selected from the group consisting of: at least 4 C atoms, at least 5 C-atoms, at least 6 C-atoms, at least 7 C-atoms, at least 8 C-atoms, at least 9 C-atoms, at least 10 C-atoms, at least 11 C-atoms, at least 12 C-atoms, at least 13 C-atoms, at least 14 C-atoms, at least 15 C-atoms, at least 16 C-atoms, at least 17 C-atoms, at least 18 C-atoms, at least 19 C-atoms and at least 20 C-atoms, an alkenyl chain with a length selected from the group consisting of: at least 4 C atoms, at least 5 C-atoms, at least 6 C-atoms, at least 7 C-atoms, at least 8 C-atoms, at least 9 C-atoms, at least 10 C-atoms, at least 11 C-atoms, at least 12 C-atoms, at least 13 C-atoms, at least 14 C-atoms, at least 15 C-atoms, at least 16 C-atoms, at least 17 C-atoms, at least 18 C-atoms, at least 19 C-atoms and at least 20 C-atoms, an alkynyl chain with a length selected from the group consisting of: at least 4 C atoms, at least 5 C-atoms, at least 6 C-atoms, at least 7 C-atoms, at least 8 C-atoms, at least 9 C-atoms, at least 10 C-atoms, at least 11 C-atoms, at least 12 C-atoms, at least 13 C-atoms, at least 14 C-atoms, at least 15 C-atoms, at least 16 C-atoms, at least 17 C-atoms, at least 18 C-atoms, at least 19 C-atoms and at least 20 C-atoms.

In another preferred embodiment, at least one membrane anchor comprise one or more aromatic rings. The aromatic rings may be heteroaromatic rings.

In yet another embodiment, at least one membrane anchor comprise a fatty acid selected from the group consisting of: butanoic acid; hexanoic acid; octanoic acid; decanoic acid; dodecanoic acid; tetradecanoic acid; hexadecanoic acid; 9-hexadecenoic acid; octadecanoic acid; 9-octadecenoic acid; 11-octadecenoic acid; 9,12-octadecadienoic acid; 9,12,15-octadecatrienoic acid; 6,9,12-octadecatrienoic acid; eicosanoic acid; 9-eicosenoic acid; 5,8,11,14-eicosatetraenoic acid; 5,8,11,14,17-eicosapentaenoic acid; docosanoic acid; 13-docosenoic acid; 4,7,10,13,16,19-docosahexaenoic acid and tetracosanoic acid.

Preferably, each membrane anchor comprise lipophilic groups selected from the groups mentioned above, e.g. two fatty acids. If only one lipophilic group is present, the membrane anchor may not be strong enough, i.e. the membrane anchor will not have a sufficient tendency to insert into the membrane. Thus, membrane anchors should neither be too strong, nor too weak.

When two lipophilic groups are present in the membrane anchor, they should preferably be positioned such as to not engage in stable hydrophobic interactions with each other. Thus, preferably, they are separated by a number of bonds selected from at least 2 bonds, at least 3 bonds, at least 4 bonds and at least 5 bonds. Preferably, the bonds are conformationally restrained.

A preferred scaffold for the membrane anchor is polyaza crown ether.

Thus, in a preferred embodiment, at least one membrane anchor comprise a polyaza crown ether with two lipophilic substituents.

Membrane anchors may be located at the 3'terminal nucleotide and at the 5'terminal nucleotide of the amphiphilic oligonucleotide.

In another embodiment, a first membrane anchor is located at least 1 nucleotide from the 5'end of the amphiphilic oligonucleotide and wherein a second membrane anchor is located at least 1 nucleotides from the 3'end of the amphiphilic oligonucleotide.

In still another embodiment, the distance between the first membrane anchor and the 5'end is selected from the group consisting of at least 2 nucleotides, at least 3 nucleotides, at least 4 nucleotides, at least 5 nucleotides and least 6 nucleotides.

In still another embodiment, the distance between the second membrane anchor and the 3'end is selected from the group consisting of at least 2 nucleotides, at least 3 nucleotides, at least 4 nucleotides, at least 5 nucleotides and least 6 nucleotides.

It may be desirable to have a distance between the terminal ends of the amphiphilic oligonucleotide and the membrane anchors to introduce steric hindrance that will prevent the membrane anchors or the lipophilic moieties of the membrane anchors from interacting. In particular, it is preferred that two lipophilic moieties of the same amphiphilic oligonucleotide are prevented from interaction, which can be obtained by having a distance between the terminal ends of the oligonucleotide and the membrane anchors.

### Lipid membranes

The lipid membranes of the invention may be mono-layered membranes or bilayered membranes.

Further, the lipid may be part of a vesicle, a micelle, a cell membrane or a cellular membrane.

Phospholipid vesicles may be prepared as outlined in the examples section.

Preferably, the size of the vesicle or micelle selected from the group consisting of: more than 20 nm, more than 50 nm, more than 75 nm, more than 100 nm, more than 150 nm and more than 200 nm.

More preferably, the size of the vesicle or micelle is selected from the group consisting of between 20 and 300 nm, between 50 and 200 nm and between 50 and 100 nm. The size of the prepared vesicle or micelle can be controlled by extrusion through polycarbonate membranes with a certain pore diameters.

In a preferred embodiment, the vesicle or micelle comprise a detectable marker selected from the group consisting of: flourophores such as 5-Carboxytetramethylrhodamine, 5-FAM (5-Carboxyfluorescein), 7-Amino-4-methylcoumarin, Bodipy, Fluorescein, Rhodamine 6G, SYTO, semiconductor quantum dots such as CdS, CdSe, CdTe, HgTe, InP, InAs (1 - 100 nm), metal nanoparticles such as Au, Ag (1 - 100 nm) and magnetic nanoparticles such as Fe3O4, FeO, Fe2O3 (5 - 200 nm).

In a preferred embodiment, the detectable marker resides within the vesicle or micelle.

Detectable markers are introduced to improve the sensitivity of detecting oligonucleotide-lipid membrane architectures. The markers may be introduced by extrusion of vesicles in buffer containing the respective marker. Non-introduced markers may be removed by purification steps.

### Target nucleic acid

The target nucleic acid may be a synthetic nucleic acid, such as a RNA or DNA oligonucleotide.

In a preferred embodiment, the target nucleic acid is derived from biological material and selected from the group consisting of, an mRNA, microRNAs, rRNA, tRNA and chromosomal DNA and a PCR product. The phrase "derived from biological material" intended to mean that the target nucleic acid is purified or separated from biological material. Nucleic acids of biological material may also be manipulated by e.g. PCR amplification or other means such as to provide a nucleic acid that is still derived from biological material.

### Detection and quantification

Disclosed are methods for detecting the presence of a particular nucleic acid, e.g. a nucleotide sequence of a certain bacteria or virus. Thus, the method may be used for detecting a particular bacteria or virus or other micro organisms in a sample obtained from a mammal.

The method may also be used for detecting a single nucleotide polymorphism (SNP) in a mammal, such as a human. As mentioned above and illustrated in the examples section, the present method provides very specific base pairing and is consequently well suited for detection of SNPs.

Oligonucleotide-lipid membrane aggregation may be detected by visual inspection of the sample comprising the amphiphilic oligonucleotide, the lipid membrane and the target nucleic acid.

The method of the invention is used for quantifying the presence of a particular nucleic acid. In this embodiment, the degree of oligonucleotide-lipid membrane aggregation may be monitored by measuring optical density of the sample comprising the amphiphilic oligonucleotide, the lipid membrane and the target nucleic acid. The measured optical density may be compared to a standard curve prepared by measurements on oligonucleotide-lipid membrane aggregation using predetermined amounts of a target nucleic acid.

Detection or quantification may also involve light-scattering techniques or UV-spectroscopy.

### Separation and purification

The method of constructing oligonucleotide-lipid membrane architectures may also be used for separating the target nucleic acid from other molecules such as other nucleic acids. This may be done, because hybridisation of the target nucleic acid to the amphiphilic oligonucleotide in the presence of a lipid membrane can lead to lipid membrane aggregation. The resulting lipid membrane aggregate can be separated from other molecules in solution e.g. by way of filtration or centrifugation. After separation, the target nucleic acid may be released from the lipid membranes e.g. denaturing agents (high ph), heating, enzymatic cleavage or photocleavage. The target nucleic acid may also be released as a complex with the amphiphilic oligonucleotide by releasing the membrane anchors from the amphiphilic oligonucleotide, e.g. by enzymatic cleavage or photocleavage.

### Immobilisation

In a preferred embodiment of the method of constructing oligonucleotide-lipid membrane architectures, the target nucleic acid is an oligonucleotide that has been or can be immobilised on a solid support.

In another embodiment, the amphiphilic oligonucleotide has been or can be immobilised on a solid support.

Immobilisation may be done by way of a capture oligonucleotide immobilized on the solid support. Immobilisation may also be done by way of a capture group introduced into the target nucleic acid or the amphiphilic oligonucleotide. Exemplary capture groups are amine-groups and carboxylic groups that may be coupled to each other by the use of NHS and EDC. Other capture groups are e.g. antigens, antibodies, biotin etc.

In a preferred embodiment, the solid support is a microarray. When the solid support is a microarray, it may be desirable at least one of the membrane anchors inserts into the lipid membrane irreversibly under the conditions employed. Alternatively, two membrane anchors may be juxtaposed to give a bivalent membrane anchor that inserts irreversibly into a lipid membrane. In such an embodiment, different capture groups may be used for different areas of the microarray allowing directed immobilisation (micropatterning) on the microarray. Preferably, the capture groups in this embodiment are oligonucleotides.

### Examples

### Example 1. Materials & Methods

### Synthesis of building blocks

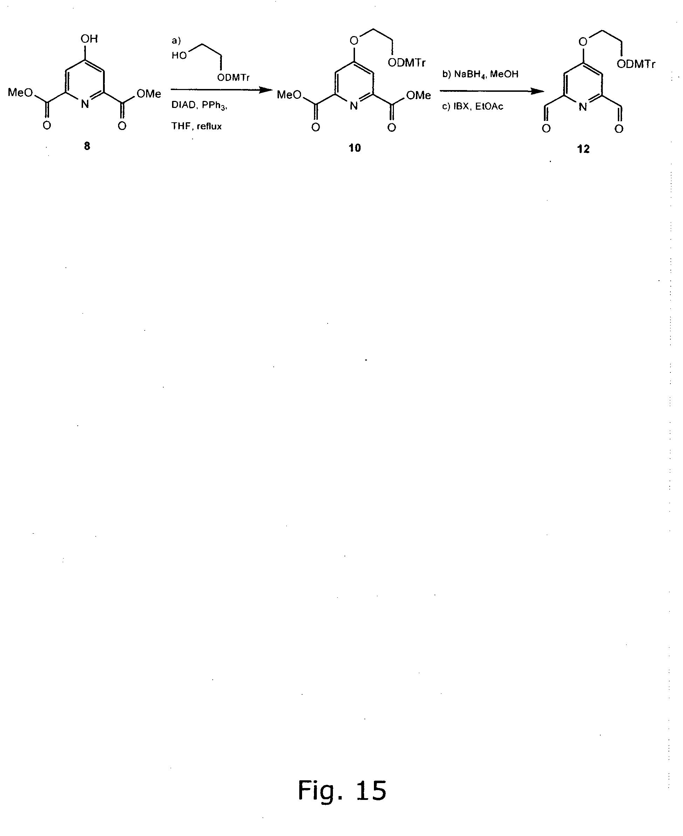

### Se fig. 14 and 15 for outline.

Synthesis of N-[2-(2-hydroxyethoxy)ethyl]hexadecanamide (2a) - General Procedure: A solution of 2-(2-aminoethoxy)-ethanol (8.21 g, 78 mmol) in water (70 ml) and THF (210 ml) was cooled to 5°C (ice bath) and MgO (15.71 g, 390 mmol) was added followed by 30 min stirring. Palmitoylchloride (21.43 g, 78 mmol) dissolved in THF (70 ml) was slowly added to the slurry keeping the temperature of the reaction mixture below 10°C. After continous rapid stirring for 2 h at 5°C the reaction mixture was filtered and the filtrate evaporated to dryness affording 2a (26.78 g, 100 %). 1H-NMR: δ (ppm) = 0.85 (t, 3H, CH3), 1.15-1.38 (m, 24H, CH2), 1.62 (m, 2H, CH2), 2.18 (bs, 1H, OH), 2.18 (m, 2H, CH2), 3.48 (m, 2H, CH2), 3.58 (m, 4H, CH2), 3.75 (m, 2H, CH2), 5.86 (bs, 1H, NH). 13C-NMR: δ (ppm) = 14.23, 22.80, 25.88, 29.47, 29.50, 29.64 29.78 29.81 32.04 36.89, 39.29, 61.83, 70.14, 72.37, 173.67. MALDI-MS: m/z calcd. for C20H41NO3 [M+Na]+: 366.2979; found: 366.2970.

Synthesis of (3a,8a,9b,10a,13a,14b,20S)-N-[2-(2-hydroxyethoxy)ethyl]cholest-5-ene-3-carboxamide (2b): Dry column FC (CHCl3:THF:HOAc-9:1:0.1) afforded 2b (9.56 g, 74 %). 1H-NMR: δ (ppm) = 1H-NMR: δ (ppm) = 0.67 (bs, 6H, CH3), 0.85-2.52 (m, 44H, CH, CH2, CH3), 3.44-3.47 (m, 2H, NCH2), 3.55-3.60 (m, 4H, OCH2), 3.73-3.76 (m, 2H, HOCH2), 5.29 (m, 2H, CH), 5.98 (m, 1H, NH). 13C-NMR: δ (ppm) = 11.98, 18.85, 19.55, 20.99, 22.69, 22.94, 23.97, 24.39, 25.93, 28.13, 28.36, 31.91, 32.03, 35.76, 35.91, 36.32, 37.09, 39.00, 39.20, 39.64, 39.91, 42.42, 46.98, 50.48, 56.32, 56.94, 61.86, 70.12, 70.35, 120.97, 141.56, 176.09. MALDI-MS: m/z calcd for C32H55NO3 [M+Na]+: 524.4074; found: 524.4067.

Synthesis of N-[2-(2-hydroxyethoxy)ethyl]benzamidei (2c): Yield: (56.9 g, 95%). 2c was used without further purification. 1H-NMR: δ (ppm) = 3.06 (bs, 1H, OH), 3.58 (m, 2H, CH2), 3.64 (d, 4H, CH2), 3.73 (m, 2H, CH2), 7.05 (bs, 1H, NH), 7.42 (m, 3H, CH), 7.78 (m, 2H, CH). 13C-NMR: δ (ppm) = 39.9, 61.7, 70.0, 72.4, 127.2, 128.6, 131.5, 134.5, 168.0. EA: Calculated: C: 63.14%, H: 7.23%, N: 6.69%, O: 22.94% Found: C: 62.66%, H: 7.25%, N: 6.68%. MALDI-MS: m/z calcd for C11H15NO3 [M+Na]+: 232.0944; found: 232.0943.

Synthesis of 2-[2-(palmitoylamino)ethoxy]ethyl methanesulfonate (3a) - General Procedure: A solution of 2a (26.78 g, 78 mmol) in THF (200 ml) was cooled to 5°C (ice bath), NEt3 (15.76 g, 158 mmol) and mesylchloride (12.3 ml, 158 mmol) were added keeping the temperature of the reaction mixture below 10°C. After continous rapid stirring for 2 h at 5°C the reaction mixture was evaporated to dryness and redissolved in CHCl3 (300 ml) followd by a wash with brine and saturated aqueous NaHCO3 solution. The organic layer were collected and evaporated to dryness affording 3a (29.6 g, 90 %). 1H-NMR: δ (ppm) = 0.88 (t, 3H, CH3), 1.15-1.45 (m, 24H, CH2), 1.62 (m, 2H, CH2), 2.18 (m, 2H, CH2), 3.06 (s, 3H, CH3), 3.46 (m, 2H, CH2), 3.58 (m, 2H, CH2), 3.73 (m, 2H, CH2), 4.38 (m, 2H, CH2), 6.06 (bs, 1H, NH). 13C-NMR: 8 (ppm) = 14.26, 22.81, 25.87, 29.48, 29.51, 29.65, 29.81, 32.04, 36.79, 38.07, 39.09, 68.81, 68.88, 70.26, 173.56. MALDI-MS: m/z calcd for C21H43NO5S [M+Na]+: 444.2754; found: 444.2736.

Synthesis of 2-(2-{[(3a,8a,9b,10a,13a,14b,20S)-cholest-5-en-3-ylcarbonyl]amino}ethoxy)ethyl methanesulfonate (3b): Yield (11 g, 99%). 1H-NMR: δ (ppm) = 1H-NMR: δ (ppm) = 0.67 (bs, 6H, CH3), 0.85-2.52 (m, 44H, CH, CH2, CH3), 3.05 (s, 3H, CH3SO20), 3.43-3.48 (m, 2H, NCH2), 3.56-3.59 (m, 2H, OCH2), 3.71-3.74 (m, 2H, OCH2), 4.36-4.39 (m, 2H, CH3SO20CH2), 5.33 (m, 2H, CH), 6.03 (m, 1H, NH). 13C-NMR: δ (ppm) = 11.99, 18.85, 19.55, 21.00, 22.69, 22.95, 23.96, 24.40, 25.93, 28.14, 28.36, 31.93, 32.05, 35.70, 35.92, 36.32, 37.10, 38.06, 39.00, 39.65, 39.92, 42.43, 46.91, 50.45, 56.31, 56.94, 68.84, 70.25, 120.92, 141.63, 175.98. MALDI-MS: m/z calcd for C33H57NO5S [M+Na]+: 602.3850; found: 602.3852.

Synthesis of 2-(2-benzamidoethoxy)ethyl methanesulfonate (3c): FC (CH2Cl2 CH2Cl2:MeOH 50:1) afforded 3c (40.2 g, 61%). 1H-NMR: δ (ppm) = 3.00 (s, 3H, SCH3), 3.68 (s, 4H, CH2), 3.76 (m, 2H, CH2), 4.38 (m, 2H, CH2), 6.79 (bs, 1H, NH), 7.27-7.50 (m, 3H, CH), 7.82 (m, 2H, CH). 13C-NMR: δ (ppm) = 37.9, 39.7, 68.8, 70.0, 127.2, 128.6, 131.6, 134.4, 167.7. EA: Calculated: C: 50.16%, H: 5.96%, N: 4.87%, O: 27.84%, S: 11.16%. Found: C: 51.14%, H: 5.95%, N: 5.07%. MALDI-MS: m/z calcd for C12H17NO5S [M+Na]+: 310.0720; found: 310.0716.

Synthesis of 3-{bis-[2-(2-palmitoylamino-ethoxy)-ethyl]-amino}-propan-1-ol (4a) - General Procedure: A mixture of n-propanolamine (1 g, 13.3 mmol), mesylate 3a (16.84 g, 39.93 mmol), triethylamine (7.41 ml, 53.2 mmol) and molecular sieve (3 Å, 2 g) in acetonitrile (60 ml, anhydrous) was refluxed for 36 h. After filtration and removal of the solvent under reduced pressure the residue was dissolved in chloroform, and washed with saturated aqueous NaHCO3 (250 ml). After extraction with dichloromethane the combined organic layers were dried with MgSO4, filtered, and the solvent was removed under reduced pressure. Dry column FCii (CH2Cl2:MeOH=40:1) afforded 4a (5.87 g, 61 %). 1H-NMR: δ (ppm) = 0.88 (dd, 6H, CH3), 1.15-1.38 (m, 48H, CH2), 1.60-1.74 (m, 6H, CH2), 2.12-2.18 (m, 4H, NCH2), 2.66-2.78 (m, 6H, NCH2), 3.40-3.56 (m, 6H, CH2NHCO, OCH2), 3.83 (m, 2H, HOCH2), 7.23 (bs, 2H, NH). 13C-NMR: δ (ppm) = 14.22, 22.79, 25.97, 27.96, 29.25, 29.47, 29.61, 29.68, 29.81, 32.03, 36.60, 39.24, 54.70, 55.83, 64.89, 68.25, 70.31, 173.59. MALDI-MS: m/z calcd for C43H87N3O5 [M+Na]+: 748.6538; found: 748.6508.

Synthesis of (3a,17a)-N-[13-[(3a,8a,9b,10a,13a,14b,20S)-cholest-5-en-3-yl]-6-(3-hydroxypropyl)-13-oxo-3,9-dioxa-6,12-diazatridec-1-yl]cholest-5-ene-3-carboxamide (4b): Dry column FC (CHCl3→CHCl3:MeOH-80:1) afforded 4b (3.61 g, 45 %). 1H-NMR: δ (ppm) = 0.67 (bs, 6H, CH3), 0.85-2.12 (m, 90H, CH, CH2, CH3), 2.42-2.78 (m, 6H, NCH2), 3.40-3.55 (m, 12H, NCH2, OCH2), 3.80 (m, 2H, HOCH2), 5.30 (m, 2H, CH), 7.16 (m, 2H, NH). MALDI-MS: m/z calcd for C67H115N3O5 [M+Na]+: 1041.88; found: 1041.88.

Synthesis of 3-{bis-[2-(2-benzoylamino-ethoxy)-ethyl]-amino}-propan-1-ol (4c): FC (CH2Cl2:MeOH:NH3(aq. 25%) = 20:1:0 → 5:1:0.1) afforded 4c (3.65 g, 40 %). 1H-NMR: δ (ppm) = 1.63 (m, 2H, CH2), 2.67 (t, 4H, CH2), 2.72 (t, 2H, CH2), 3.58 (m, 14H, CH2), 7.38 (m, 6H, CH), 7.73 (bs, 2H, 2x NH), 7.81 (m, 4H, CH). 13C-NMR: δ (ppm) = 27.9, 40.0, 54.1, 55.8, 64.5, 68.2, 70.2, 127.3, 128.3, 131.2, 134.8, 167.9. MALDI-MS: m/z calcd for C25H35N3O5 [M+Na]+: 480.24689; found: 480.24720.

Synthesis of 3-(bis{2-[2-(hexadecylamino)ethoxy]ethyl}amino)propan-1-ol (5a)-General Procedure: A solution of 4a (5.87 g, 8.08 mmol) in THF (50 ml) was added slowly at 0°C to a solution of LiAlH4 (0.31 g, 1M solution in THF, 24.25 mmol). The reaction mixture was stirred for 1 h at 0°C, and then refluxed for 16 h. After cooling to 0°C and dilution to double volume with THF a 5% aquous solution of NaOH (20 ml) was added dropwise and the solution was stirred for additional 30 min at 0°C and 90 min at rt. Filtration, rinsing with hot THF (300 ml), removal of the solvent under reduced pressure, coevaporation with toluene and dry column FCii (CH2Cl2:MeOH:NH3(aq. 25%) -10:1:0.1) afforded 5a (4.8 g, 85 %). 1H-NMR: δ (ppm) = 0.88 (t, 6H, CH3), 1.15-1.48 (m, 54H, CH2), 1.48 (m, 4H, CH2), 1.66 (m, 2H, CH2), 2.59 (m, 4H, CH2), 2.68-2.79 (m, 10H, CH2), 3.49-3.58 (m, 8H, CH2), 3.75 (m, 4H, CH2). 13C-NMR: δ (ppm) = 14.23, 22.80, 27.55, 28.64, 29.48, 29.81, 30.26, 32.06, 49.54, 50.24, 54.09, 55.40, 63.88, 69.20, 70.71. MALDI-MS: m/z calcd for C43H91N3O3 [M+Na]+: 720.6953; found: 720.6980.

Synthesis of 3-{[2-(2-{[(3a,17a)-cholest-5-en-3-ylmethyl]amino}ethoxy)ethyl][2-(2-{[(3a,8a,9b,10a,13a,14b,20S)-cholest-5-en-3-ylmethyl]amino}ethoxy)ethyl]amino}-propan-1-ol (5b): Yield: (2.30 g, 99 %). 1H-NMR: δ (ppm) = 0.67 (bs, 6H, CH3), 0.85-2.12 (m, 90H, CH, CH2, CH3), 2.48 (m, 4H, NCH2), 2.70-2.76(m, 10H, NCH2), 3.53-3.56 (m, 8H, OCH2), 3.73-3.77 (m, 2H, CH2), 5.29 (m, 2H, CH). 13C-NMR: δ (ppm) = 12.01, 18.87, 19.63, 21.07, 22.71, 22.96, 23.99, 24.43, 27.36, 28.15, 28.39, 28.61, 32.04, 35.95, 36.35, 37.51, 37.83, 39.40, 39.66, 39.99, 42.45, 49.66, 50.56, 54.08, 55.43, 56.32, 56.70, 56.97, 63.94, 69.22, 70.57, 119.78, 142.84. MALDI-MS: m/z calcd for C67H119N3O3 [M+Na]+: 1036.9144; found: 1036.9109.
Synthesis of 3-{bis-[2-(2-benzylamino-ethoxy)-ethyl]-amino}-propan-1-ol (5c): Yield (3.40 g, 99%). 5c was used without further purification. Analytical column (CH2Cl2:MeOH:NH3(aq. 25%) - 20:1:0 → 10:1:0 → 10:1:0.1). 1H-NMR: δ (ppm) = 1.64 (m, 2H, CH2), 2.71 (m, 6H, CH2), 2.78 (t, 4H, CH2), 3.54 (m, 8H, CH2), 3.70 (t, 2H, CH2), 3.80 (s, 4H, CH2), 7.23-7.34 (m, 10H, CH). 13C-NMR: δ (ppm) = 28.4, 48.8, 53.9, 54.1, 55.4, 70.0, 69.1, 70.4, 127.1, 128.4, 128.5, 140.0. MALDI-MS: m/z: calcd for C25H39N3O3 [M+Na]+: 452.2884; found: 452.2883.

Synthesis of 3-[19-{2-[bis(4-methoxyphenyl)(phenyl)methoxy]ethoxy}-3,15-dihexadecyl-6,12-dioxa-3,9,15,21-tetraazabicyclo[15.3.1]henicosa-1(21),17,19-trien-9-yl]propan-1-ol (6a) - General Procedure: Sodium triacetoxy borohydride (1.21 g, 5.72 mmol) was added to a solution of 12 (0.71 g, 1.43 mmol) and molecular sieves (3Å, 2 g) in 1,2-dichloroethane (30 ml, anhydrous). After addition of compound 5a (1.00 g, 1.43 mmol) in 1,2-dichloroethane (30 ml, anhydrous) the reaction mixture was stirred for 48 h at rt. Then saturated sodium bicarbonate solution (30 ml) was added, and the mixture stirred for additional 2 h. After extraction with chloroform the combined organic layers were dried with MgSO4, filtered, and the solvent was removed under reduced pressure. FC (CHCl3:MeOH:NEt3 →10:1:0.1) afforded 6a (0.68 g, 41%). 1H-NMR: δ (ppm) 0.87 (m, 6H, CH3), 1.06-1.42 (m, 52H, CH2), 1.53 (m, 6H, CH2), 2.51-2.71 (m, 14H, NCH2), 3.31-3.44 (m, 8H, OCH2), 3.70-3.78 (m, 14H, CH2, OCH3), 4.18 (dd, 2H, CH2), 6.81-7.49 (m, 15H, CH). 13C-NMR: δ (ppm) = 14.27, 22.83, 27.67, 28.55, 29.50, 29.84, 32.06, 53.16, 54.32, 55.33, 56.35, 56.91, 61.20, 62.31, 64.12, 67.32, 69.10, 69.60, 86.29, 108.06, 113.23, 126.89, 127.94, 128.33, 130.21, 136.18, 144.93, 158.60, 161.37, 166.07. MALDI-MS: m/z calcd for C73H118N4O7 [M+Na]+: 1185.8893; found: 1185.8873.

Synthesis of 3-[19-{2-[bis(4-methoxyphenyl)(phenyl)methoxy]ethoxy}-3-[(3a,8a,9b,10a,13a,14b, 20S)-cholest-5-en-3-ylmethyl]-15-[(3b,17a)-cholest-5-en-3-ylmethyl]-6,12-dioxa-3,9,15,21-tetra-aza-bicyclo[15.3.1]henicosa-1(21),17,19-trien-9-yl]propan-1-ol (6b): FC (CHCl3:MeOH:NEt3 →20:1:0.1) afforded 6b (1.49 g, 46 %). 1H-NMR: δ (ppm) = 0.67 (bs, 6H, CH3), 0.85-2.22 (m, 90H, CH, CH2, CH3), 2.42-2.67 (m, 14H, NCH2), 3.32-3.44 (m, 8H, OCH2), 3.70-3.78 (m, 14H, CH2, NCH2, OCH3), 4.18 (m, 2H, CH2), 5.30 (m, 2H, CH), 6.81-7.49 (m, 15H, CH). 13C-NMR: δ (ppm) = 11.99, 18.85, 19.66, 21.06, 22.70, 22.95, 23.94, 24.41, 24.53, 27.72, 28.14, 28.37, 28.53, 32.02, 35.92, 36.32, 37.64, 38.25, 38.39, 39.55, 39.65, 39.96, 42.20, 50.61, 53.54, 54.38, 55.30, 56.27, 56.43, 56.95, 61.76, 62.33, 63.59, 64.14, 67.24, 69.07, 69.45, 86.28, 108.02, 113.21, 119.76, 126.88, 127.94, 128.34, 130.19, 136.19, 143.12, 144.89, 158.59, 161.45, 165.99. MALDI-MS: m/z calcd for C97H146N4O7 [M+Na]+: 1502.1084; found: 1502.1050.

Synthesis of 3-[3,15-dibenzyl-19-{2-[bis(4-methoxyphenyl)(phenyl)methoxy]ethoxy}-6,12-dioxa-3,9,15,21-tetraazabicyclo[15.3.1]henicosa-1(21),17,19-trien-9-yl]propan-1-ol (6c): FC (CHCl3:MeOH:NEt3 →10:1:0.1) afforded 10 (1.36 g, 65%). 1H-NMR: δ (ppm) 1.60 (m, 2H, CH2), 2.56 (t, 4H, CH2), 2.62 (t, 2H, CH2), 2.77 (t, 4H, CH2), 3.36 (t, 4H), 3.46 (m, 6H, CH2), 3.68-3.80 (m, 16H, CH2, OCH3), 4.16 (t, 2H, CH2), 6.81-6.84 (m, 6H, CH), 7.20-7.49 (m, 19H, CH), 13C-NMR: δ (ppm) = 28.5, 52.9, 54.4, 55.3, 56.3, 60.5, 60.6, 62.3, 64.1, 67.2, 69.2, 69.5, 86.3, 108.0, 113.2, 126.9, 127.1, 127.9, 128.3, 128.4, 129.0, 130.2, 136.2, 139.6, 144.9, 158.6, 160.9, 166.0. MALDI-MS: m/z calcd for C55H66N4O7 [M+Na]+: 917.4824; found: 917.4817.

Synthesis of 3-[19-{2-[bis(4-methoxyphenyl)(phenyl)methoxy]ethoxy}-3,15-dihexadecyl-6,12-dioxa-3,9,15,21-tetraazabicyclo[15.3.1]henicosa-1(21),17,19-trien-9-yl]propyl-2-cyanoethyldiisopropyl-amidophosphite (7a) - General Procedure: 2-cyanoethyl diisopropyl-amidochloridophosphite (54.4 mg, 0.206 mmol) is added slowly at 0°C to a solution of 6a (200 mg, 0.171 mmol), and diisopropylethylamine (53.3 mg, 0.412 mmol) in dichloromethane (5 ml, anhydrous). After stirring for 1h at 0°C the reaction mixture was washed with saturated sodium bicarbonate solution and extracted with dichloroethane. The combined organic layers were dried with MgSO4, filtered, and solvents removed under reduced pressure. FC (EE:NEt3 - 10:0.2) afforded 7a (141.5 mg, 60%). 1H-NMR: δ (ppm) = 0.87 (m, 6H, CH3), 1.05-1.42 (m, 68H, CH2), 1.48-1.69 (m, 6H, CH2), 2.50-2.70 (m, 16H, CH2CN, NCH2), 3.25-3.81 (m, 26H, OCH2, OCH3, NCH2, CH2), 4.18 (dd, 2H, OCH2), 6.81-7.55 (m, 15H, CH). 13C-NMR: δ (ppm) 14.26, 20.42, 20.51, 22.82, 24.68, 24.71, 24.77, 24.80, 27.67, 29.00, 29.10, 29.50, 29.83, 32.06, 43.03, 43.19, 53.01, 53.35, 54.46, 55.32, 57.01, 58.24, 58.50, 61.24, 61.92, 62.15, 62.29, 67.33, 69.58, 69.80, 86.29, 108.09, 113.23, 126.89, 127.94, 128.33, 130.20, 136.19, 144.93, 158.61, 161.46, 166.00. 31P-NMR: δ (ppm) = 148.32. HR-ESI-MS: m/z calcd for C82H135N6O8P [M+2H]2+: 682.5112; found: 682.5120.

Synthesis of 3-[19-{2-[bis(4-methoxyphenyl)(phenyl)methoxy]ethoxy}-3-[(3a,8a,9b,10a,13a,14b,20S)-cholest-5-en-3-ylmethyl]-15-[(3b,17a)-cholest-5-en-3-ylmethyl]-6,12-dioxa-3,9,15,21-tetraazabicyclo-[15.3.1]henicosa-1(21),17,19-trien-9-yl]propyl-2-cyanoethyldiiso-propylamidophosphite (7b): FC (EE:NEt3 - 10:0.2) afforded 7b (255 mg, 56 %). 1H-NMR: δ (ppm) = 0.67 (bs, 6H, CH3), 0.85-2.22 (m, 96H, CH, CH2, CH3), 2.42-2.67 (m, 16H, NCH2), 3.24-3.88 (m, 26H, CH2, NCH, NCH2, OCH2, OCH3), 4.18 (m, 2H, CH2), 5.30 (m, 2H, CH), 6.81-7.49 (m, 15H, CH). 13C-NMR: δ (ppm) = 12.01, 18.86, 19.57, 19.67, 20.43, 20.52, 21.08, 21.46, 22.70, 22.96, 23.95, 24.42, 24.71, 24.78, 27.80, 28.15, 28.38, 29.05, 29.15, 32.04, 35.93, 36.33, 37.65, 38.28, 38.42, 39.57, 39.66, 39.98, 42.43, 43.03, 43.29, 50.63, 53.03, 53.79, 54.53, 56.28, 56.96, 58.26, 58.51, 61.88, 62.16, 62.34, 63.70, 67.25, 69.51, 69.79, 86.29, 108.11, 113.22, 119.77, 126.89, 127.95, 128.34, 130.19, 136.20, 143.13, 144.90, 158.59, 161.56, 165.97. 31P-NMR: δ (ppm) = 148.30, 149.38 (< 5 %).

Synthesis of 2-cyanoethyl-3-[3,15-dibenzyl-19-{2-[bis(4-methoxyphenyl)-(phenyl)methoxy]-ethoxy}-6,12-dioxa-3,9,15,21-tetraazabicyclo[15.3.1]henicosa-1(21),17,19-trien-9-yl]propyldiiso-propyl-amidophosphite (7c): FC (EE:NEt3 - 10:0.2) afforded 7c (305 mg, 83%). 1H-NMR: δ (ppm) = 1.16 (t, 12H, CH3), 1.68 (m, 2H, CH2), 2.56 (m, 8H, CH2), 2.76 (t, 4H, CH2), 3.34 (t, 4H, CH2), 3.44 (m, 6H, CH2), 3.53-3.81 (m, 20H, CH2), 4.15 (t, 2H, CH2), 6.82 (m, 6H, CH), 7.21-7.40 (m, 19H, CH). 13C-NMR: δ (ppm) = 20.49, 24.72, 24.80, 29.10, 43.05, 43.21, 52.94, 53.05, 54.56, 55.35, 58.38, 60.58, 60.72, 62.06, 62.28, 67.27, 69.46, 69.83, 86.33, 108.06, 113.25, 126.93, 127.17, 127.96, 128.34, 128.43, 129.05, 130.21, 136.17, 139.59, 144.91, 158.63, 161.00, 165.96. 31P-NMR: δ (ppm) = 148.3 (s).

Synthesis of 4-[2-(4,4'-dimethoxytrityl)-oxy-ethoxy]-pyridine-2,6-dicarboxylic acid dimethyl ester (10): Ethyleneglycol (39.7 g, 640 mmol, 36 ml) and N,N-dimethoxy-4-amino-pyridine (977 mg, 8 mmol) were dissolved in dichloromethane (200 ml, anhydrous) and cooled to 0°C. Under Ar-atmosphere diisopropylethylamine (24.8 g, 192 mmol, 33.5 ml) and then 4,4'-dimethoxytritylchloride (54.2 g, 160 mmol) in dichloromethane (500 ml, anhydrous) were added slowly. After 3 h stirring at rt the solvent was removed under reduced pressure. FC (CHCl3: NEt3 - 20:0.1) afforded 2-(4,4'-dimethoxytrityl)-ethyleneglycol (42.2 g, 72%) 9. 1H-NMR: δ (ppm) = 3.25 (t, 3JH,H=4.7 Hz, 2H, CH2), 3.73 (t, 3JH,H=4.7 Hz, 2H, CH2), 3.78 (s, 6H, CH3), 6.82 (m, 4H, CH), 7.23 - 7.34 (m, 7H, CH), 7.44 (d, 2H, CH), 13C-NMR: δ (ppm) = 55.3, 62.5, 64.7, 86.2, 113.3, 126.9, 128.0, 128.3, 130.2, 136.2, 145.0, 158.6. MALDI-MS m/z: calcd for C23H24O4 [M+Na]+: 387.1567; found: 387.1576. Diisopropyl azodicarboxylate (21.25 g, 105.12 mmol, 20.28 ml) was added under Ar-atmosphere at 0°C and in the dark to a solution of triphenylphosphine (27.57 g, 105.12 mmol) in THF (150 ml, anhydrous). After stirring at 0°C for 30 min, first 2-(4,4'-dimethoxytrityl)-ethyleneglycol (31.92 g, 87.59 mmol) in THF (100 ml, anhydrous) and then 4-(hydroxy-ethoxy)-pyridine-2,6-dicarboxylic acid dimethyl ester hydrochlorid 8 (21.50 g, 87.05 mmol) in THF (200 ml, anhydrous) were added at 0°C. After reflux for 20 h the solvent was removed under reduced pressure. Dry column (toluene:CHCl3: NEt3 - 5:4:0.1) afforded 10 (37.29 g, 77%). 1H-NMR: δ (ppm) = 3.50 (t, 2H, CH2), 3.79 (s, 6H, CH3), 4.02 (s, 6H, CH3), 4.29 (t, 2H, CH2), 6.83 (m, 4H, CH), 7.20-7.35 (m, 7H), 7.45 (d, 2H), 7.86 (s, 2H, CH). 13C-NMR: δ (ppm) = 53.4, 55.3, 62.0, 68.6, 86.6, 113.3, 114.8, 127.0, 128.0, 128.2, 130.2, 135.9, 144.7, 149.9, 158.7, 165.3, 167.2. MALDI-MS m/z: calcd for C32H31NO8 [M+Na]+: 580.1942; found: 580.1937.

Synthesis of 4-[2-(4,4'-dimethoxytrityl)-oxy-ethoxy]-2,6-bis-hydroxymethyl-pyridine (11): NaBH4 (21 g, 552 mmol) was added at 0°C in small portions to a solution of the crude dimethyl ester 10 (38.1 g, max 43 mmol) in methanol (300 ml, anhydrous). After stirring for 6 h at rt, first the reaction mixture was carefully diluted with water (50 ml), then NaHCO3 (sat. aqu. solution, 50 ml) was added slowly. Some of the methanol (∼200 ml) was removed under reduced pressure. The residue was extracted with chloroform, and the combined organic layers were washed with saturated sodium bicarbonate solution and water. After drying with MgSO4, filtration and removal of the solvent under reduced pressure, the crude was purified with FC. FC (CHCl3:MeOH:Et3N = 50:1:0.1 → 10:1:0.1) afforded 11 (16.87 g, 78% over 2 steps). 1H-NMR: δ (ppm) = 3.44 (t, 2H, CH2), 3.78 (s, 6H, CH3), 4.17 (t, 2H, CH2), 4.69 (s, 4H, CH2), 6.74 (s, 2H, CH), 6.81 (m, 4H, CH), 7.20 - 7.35 (m, 7H, CH), 7.44 (d, 2H, CH). 13C-NMR: δ (ppm) = 55.4, 62.1, 64.6, 67.7, 86.4, 105.9, 113.3, 127.0, 128.0, 128.3, 130.2, 136.0, 144.8, 158.6, 160.5, 166.6. MALDI-MS: m/z calcd for C30H31NO6 [M+Na]+: 524.2044; found: 524.2021.

Synthesis of 4-[2-(4,4'-dimethoxytrityl)-oxy-ethoxy]-pyridine-2,6-dicarbaldehyde (12): To a solution of dialcohol 11 (234 mg, 0.467 mmol) and pyridine (122 mg, 1.54 mmol) in ethylacetate (anhydrous, 5 ml) was added IBX (432 mg, 1.54 mmol) at 80°C. After stirring for 90 min at 80°C the reaction mixture was cooled down to 0°C, filtered, washed with ethylacetate, and the solvent was removed under reduced pressure. FC (CHCl3:MeOH:NEt3 - 20:1:0.1) afforded 12 (200 mg, 86%). 1H-NMR: δ (ppm) = 3.51 (t, 2H, CH2), 3.78 (s, 6H, CH3), 4.30 (t, 2H, CH2), 6.82 (d, 4H, CH), 7.25-7.33 (m, 7H, CH), 7.43 (d, 2H, CH), 7.68 (s, 2H, CH), 10.11 (s, 2H, CH). 13C-NMR: δ (ppm) = 55.4, 61.9, 68.8, 86.6, 111.8, 113.3, 127.0, 128.0, 128.2, 130.2, 135.8, 144.7, 154.9, 158.6, 167.2, 192.5. MALDI-MS: m/z calcd for C30H27NO6 [M+Na]+: 520.1731; found: 520.1721.

### Materials:

1-palmitoyl-2-oleoyl-sn-glycero-3-phosphocholine (POPC, lyophilized powder) was purchased from Avanti Polar Lipids and 1,1'-dioctadecyl-3,3,3',3'-tetramethylindocarbocyanine perchlorate (DiI-C18) and 3,3'-dioctadecyloxacarbocyanine perchlorate (DiO-C18) from Invitrogen/Molecular Probes. 2,2'-(1,8-Dihydroxy-3,6-disulfonaphthylene-2,7-bisazo)bisbenzene arsonic acid (arsenazo III) and all other chemicals were obtained from Sigma in the purest form available. Ultrapure Milli-Q water was used in all experiments. 4-(2-Hydroxyethyl)piperazine-1-ethanesulfonic acid (HEPES) buffer (10 mM HEPES, 110 mM Na+) was prepared by mixing appropriate amounts of HEPES, HEPES sodium salt and NaCl followed by correction of pH to 7.0. The chemicals for DNA synthesis were purchased from Glen Research. All solvents (HPLC grade) and reagents were purchased commercially and used without further purification. Muscovite mica (75 mm x 25 mm x 200 µm sheets) was from Plano GmbH, Germany. Mica sheets of 10 mm x 10 mm were preglued onto round (0.17 mm, 024 mm) microscope coverslips using a transparent and biocompatible silicone glue (MED-6215, Nusil Technology, Santa Barbara, CA). Immediately prior to use, the mica was cleaved with a knife leaving a thin and highly transparent mica film on the coverslip.

### Measurement of transition temperatures:

Transition analyses were carried out on a Perkin Elmer UV/VIS spectrometer Lambda 30 with a PTP-6 (Peltier Temperature Programmer) device by using PE TempLab 2.0 and UV Winlab 2.8. Melting temperatures (Tm, in °C) were determined as a first derivative of thermal denaturation curves, which were obtained by recording absorbance at 260 nm as a function of temperature at a rate of 0.5°C min-1 for measurements with liposomes and with 1°C min-1 for measurements without liposomes. The solutions were heated to 90 °C, maintained for 5 min at this temperature, and then gradually cooled before performing thermal denaturation experiments. All melting temperatures are reported with an uncertainty of ±1 °C, as determined from multiple experiments.

Experimental procedure for liposome assembly: In a typical experiment we use POPC (1-palmitoyl-2-oleoyl-sn-glycero-3-phosphocholine) liposomes with an average diameter of 65 nm ± 15 nm and a lipid concentration of 10 mM, prepared by extrusion through 50 nm filters, a final concentration of 0.5 mM is used for all experiments. Addition of lipophilic probe DNA in HEPES buffer solution (pH 7.0, 110 mM Na+, 62 nM probe strand) followed by addition of the target sequence initiates assembly. The total sample volume was 1 ml. Experiments with lower target concentrations (target concentration < 100 nM) produce stable aggregates as observed by light scattering, but without macroscopic precipitation, even after prolonged time (24 h).

### Experiments using serum and yeast cell extracts:

10 µl of FBS serum pH 6.9-7.8 pH was added to the solution of probe and target strand followed by addition of Hepes buffer to a total volume of 1 ml according to the standard procedure for liposome assembly.

10 µl of yeast cell extract was added to the solution of probe and target strand followed by addition of Hepes buffer to a total volume of 1 ml according to the standard procedure for liposome assembly.

### Preparation of POPC liposomes:

POPC was suspended in aqueous HEPES buffer at 10 mM lipid concentration and stirred to a uniform milky solution. Liposomes were prepared by repeated extrusion (10 times) through double polycarbonate filters with a 50 nm pore size using compressed N2 (30 bar) and a LIPEX™ Extruder from Northern Lipids. DiO-C18-labeled liposomes were prepared by co-solubilization of POPC and DiO-C18 in CHCl3 followed by evaporation of CHCl3 and hydration in HEPES buffer prior to extrusion.

### Preparation of supported membranes:

Supported membranes were prepared by hydration of spincoated lipid films as described previously. The procedure consists of the following steps: To prepare a dry spincoated POPC film on mica a stock solution of 10 mM POPC containing 0.7 % DiO-C18 in hexane/methanol (97:3 volume ratio) was used. A droplet (30 µL) of this lipid stock solution was then applied to freshly cleaved mica with a size of 10 mm x 10 mm and immediately thereafter spun on a Chemat Technology, KW-4A spin-coater at 3000 rpm for 40 s. The sample was placed under vacuum for 10-15 hours to ensure complete evaporation of solvents. The dry spin coated film was subsequently hydrated by immersing the sample in HEPES buffer followed by heating in an oven at 55°C for 1 hour. The sample surface was flushed with HEPES buffer at 55°C which left a single and uniform membrane on the mica surface. After this, the buffer was replaced 10 times to remove membrane fragments in solution.

### Experimental conditions for DNA controlled tethering of liposomes to a supported POPC membrane:

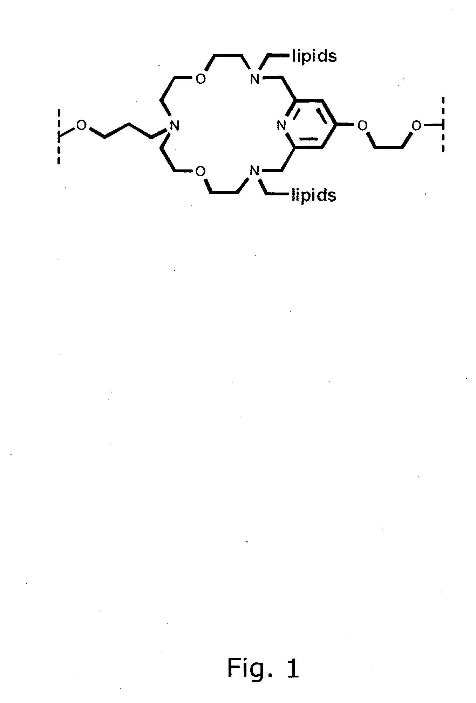

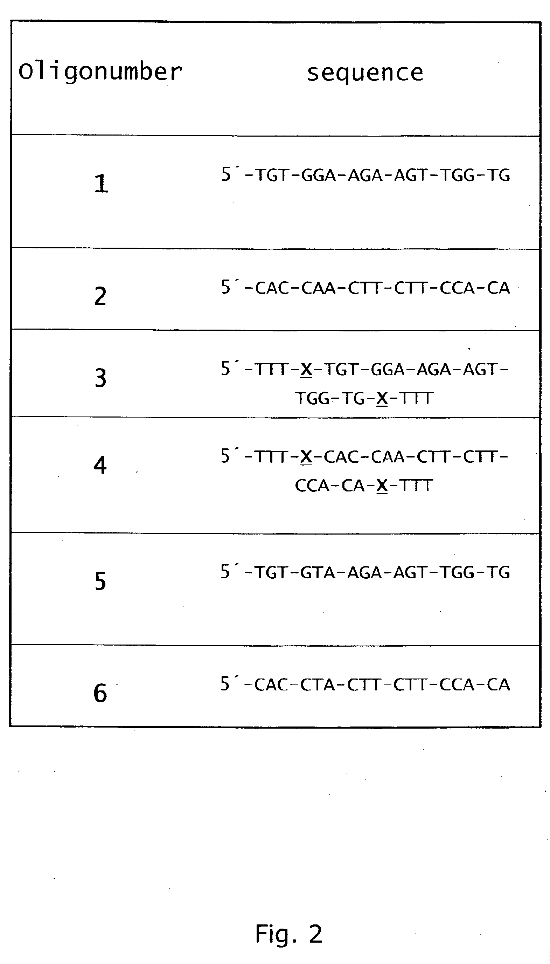

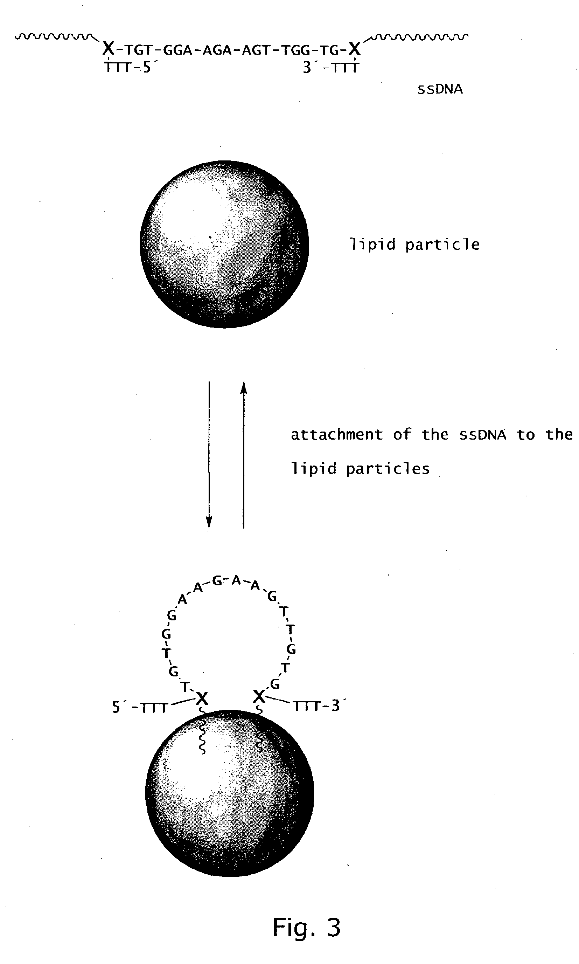

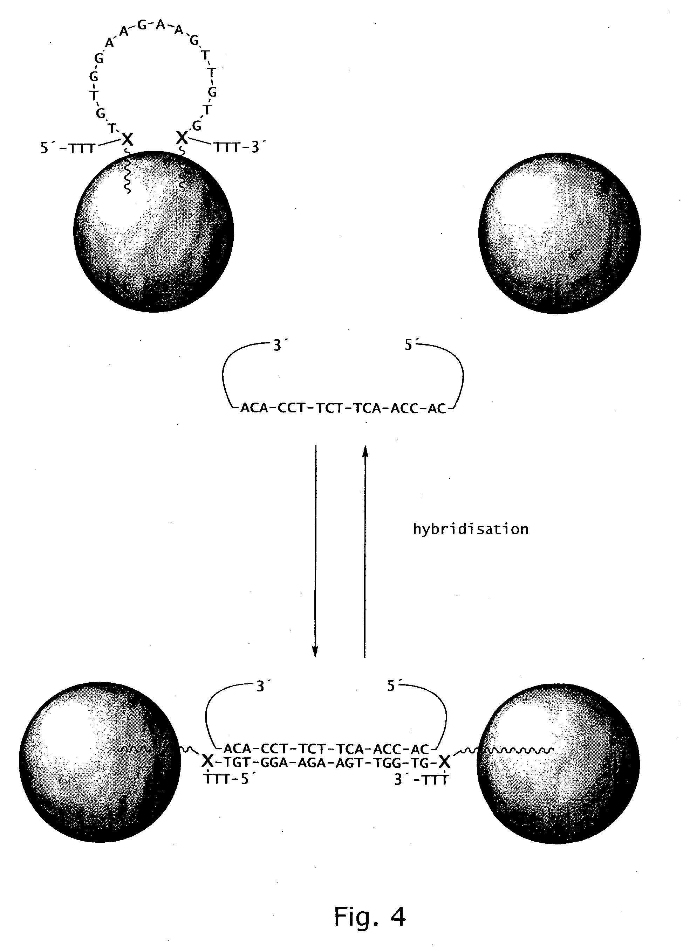

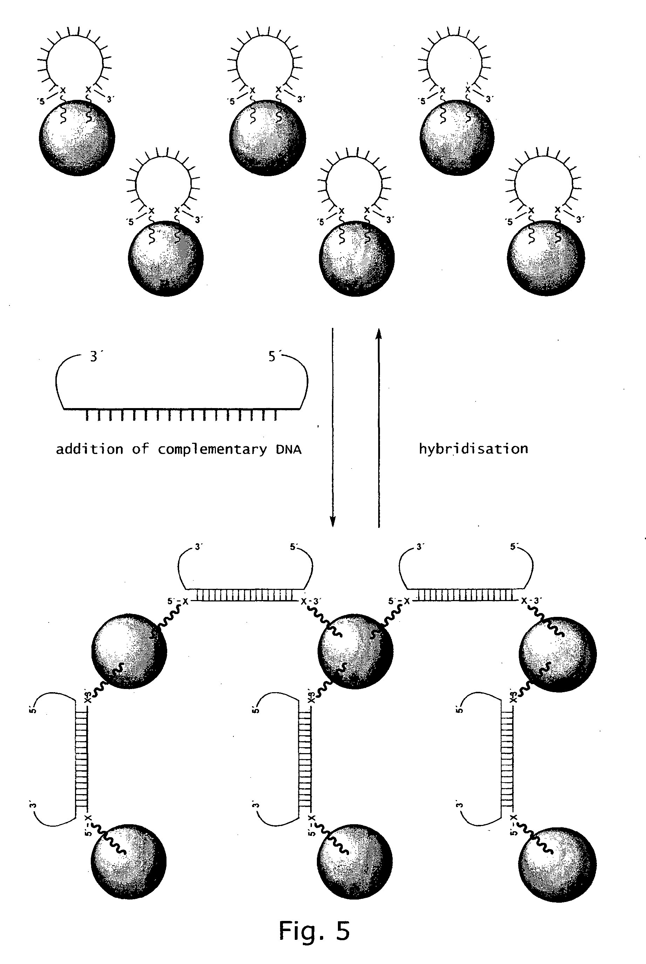

In a typical experiment for DNA controlled tethering of liposomes to a surface, a supported POPC membrane containing fluorescent dye (DiO-C18, 0.5%) was first prepared on a mica surface by spincoating according to our recent protocol. Successful membrane formation was confirmed by independent fluorescence imaging. Subsequently, the membrane was exposed to probe DNA which couples to the membrane by insertion of the alkyl chains. Liposomes labeled with a second fluorophore (DiI-C18, 0.5%) were subsequently added, but as expected no adhering to the supported membrane was observed. Finally, the target strand was added leading to duplex formation and tethering of liposomes to the surface within a time of 1-2 hours (fig. 3). However, the assembly occurs to a large extend (ca. 90%) within the first 15 min.

### Oligonucleotide synthesis and MALDI-TOF analysis of DNA-conjugates:

Standard procedures for automated DNA synthesis have been used except pyridinium hydrochloride as activator reagent and coupling times of 20 min. Incorporation of monomers into oligodeoxynucleotides (ONs) was performed on a 0.2 µmol scale and was followed by purification of the ONs by HPLC as previously described. MALDI-TOF analysis was performed on a Voyager Elite Biospectrometry Research Station from PerSeptive Biosystems.

### Fluorescence Microscopy:

Epi-fluorescence microscopy of supported membranes coupled to liposomes was performed with the sample placed with the lipid side facing up in a custom made microscope chamber (2 ml volume) and placed on a Nikon TE2000 inverted microscope using an oil immersion objective (Plan Apo, 60X NA=1.4, Nikon). Fluorescence excitation was done using a Xenon lamp with monochromator (Polychrome V, Till Photonics, Gräfelfing, Germany) as well as G-2A (DiI) and B-2A (DiO) filtercubes from Nikon. Images were recorded with a high sensitivity CCD camera (Sensicam em, 1004 x 1002 pixels, PCO-imaging, Kelheim, Germany) and operated with Camware software (PCO). The experiment was performed in a total volume of 1 ml HEPES buffer (10 mM HEPES, 110 mM Na+, pH 7.0) with a concentration of 100 nM for each strand (DNA 3'-ACACCTTCTTCAACCAC : 5'-TTTXTGTGGAAGAAGTTGGTGXTTT). Successful membrane formation was confirmed by independent fluorescence imaging. Thereafter liposomes labeled with a second fluorophor (DiI-C18, 0.5%) and probe strand were added, but without liposomes adhering to the supported membrane. Finally, the target strand was added leading to duplex formation and attachment of liposomes to the surface within a time of 1-2 hours. However an almost complete attachment (immobilization on the supported membrane) was observed already after 15 min. Thermal denaturation of liposome aggregates could thereby be visualized directly by fluorescence. Heating to above the denaturation temperature of the duplex (Tm = 46°C), led to complete release of the attached liposomes into solution and re-aggregation upon cooling below the Tm.

### Preparation of Phospholipid Vesicles.

The phospholipids used were 1,2-dimyristol- sn-phosphatidylcholine (DMPC) and 1-palmitoyl-2-oleoyl- sn-phosphatidylcholine (POPC) (Northern Lipids, Vancouver BC); the lipids were obtained in powder form. For each sample, a single variety of phospholipid was hydrated using purified water from a Milli-Q Plus water purification system (Millipore, Bedford MA), in ratios of 1-200 mg of phospholipids per milliliter of water. Water from a Milli-Q Plus filtration system was used to keep the concentration of contaminants in the vesicle solution negligible. This ensures that the vesicles will swell to spherical shapes after extrusion. The pre-extruded vesicle suspension was then extruded through two polycarbonate membranes with nominal pore diameters of 50, 100, or 200 nm using an extruder (Lipex Biomembranes, Vancouver, BC) by applying a pressure gradient as provided by prepurified, compressed N2 gas. The area of the membrane available for extrusion was 4.15 cm2. Water from an external water bath was circulated through the extruder in order to control the extrusion temperature. DMPC vesicles were extruded at 40 °C and POPC vesicles were extruded at 25 °C so that both lipids were at temperatures well above their gel-transition temperatures. The vesicle suspension was re-extruded a minimum of 10 times or until the average flow rate became constant on consecutive extrusions. The average flow rate of the extruded suspension was measured. The size of vesicles can be controlled e.g. by choosing different polycarbonate membranes with nominal pore diameters of 50, 100, or 200 nm using an extruder (Lipex Biomembranes, Vancouver, BC) by applying a pressure gradient as provided by prepurified, compressed N2 gas.

### Example 2

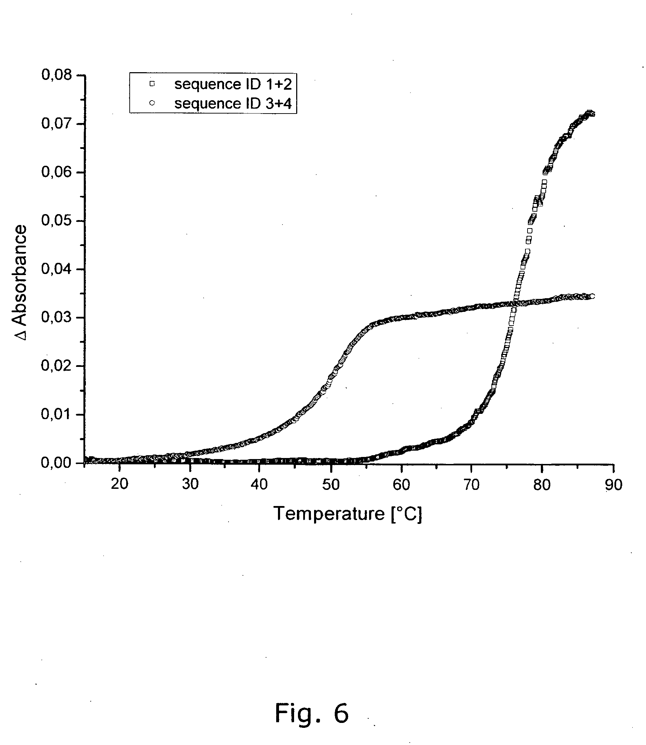

Thermal denaturation curves was recorded in a UV experiment at 260 nm wavelength showing hybridisation of sequence ID1 and ID2 compared to the hybridisation of sequence ID3 and ID4 (fig. 6)

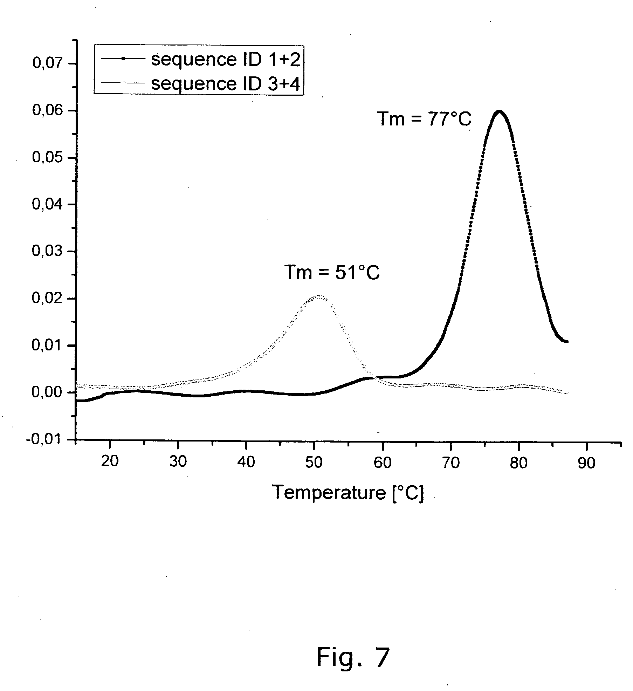

First derivatives of the thermal denaturation curves recorded in a UV experiment at 260 nm wavelength showing hybridisation of sequence ID1 and ID2 compared to the hybridisation of sequence ID3 and ID4 (fig. 7)

### Example 3

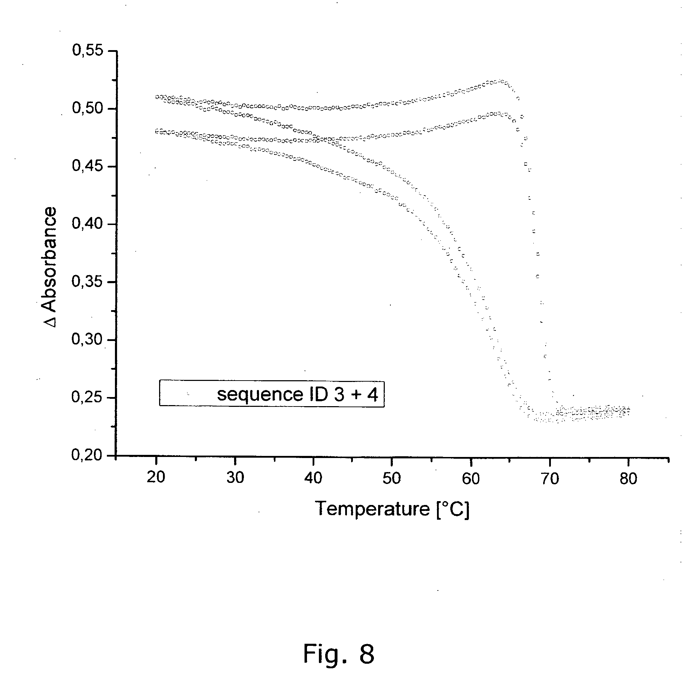

Thermal denaturation curves recorded in a UV experiment at 260 nm wavelength showing hybridisation of sequence IDI and ID2 compared to the hybridisation of sequence ID3 and ID4 and run in two consecutive cycles from 15°C to 80°C. (fig. 8). The results show the reproducibility of the assembly and disassembly process of the bilayers used (vesicles).

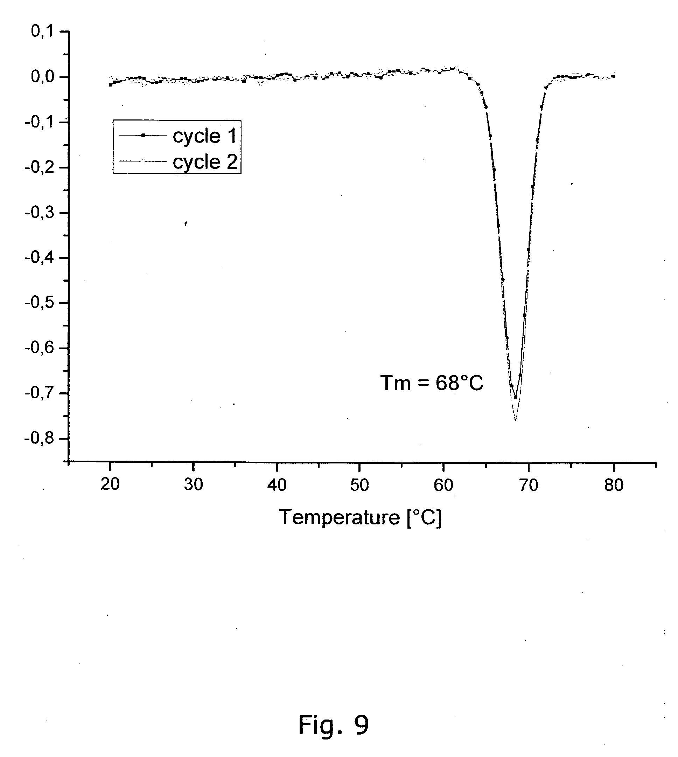

First derivative of the thermal denaturation curves recorded in a UV experiment at 260 nm wavelength showing hybridisation of sequence ID1 and ID2 compared to the hybridisation of sequence ID3 and ID4 and run in two consecutive cycles from 15°C to 80°C (fig. 9). The results again show the reproducibility of the assembly and disassembly process of the bilayers used (vesicles).

### Example 4

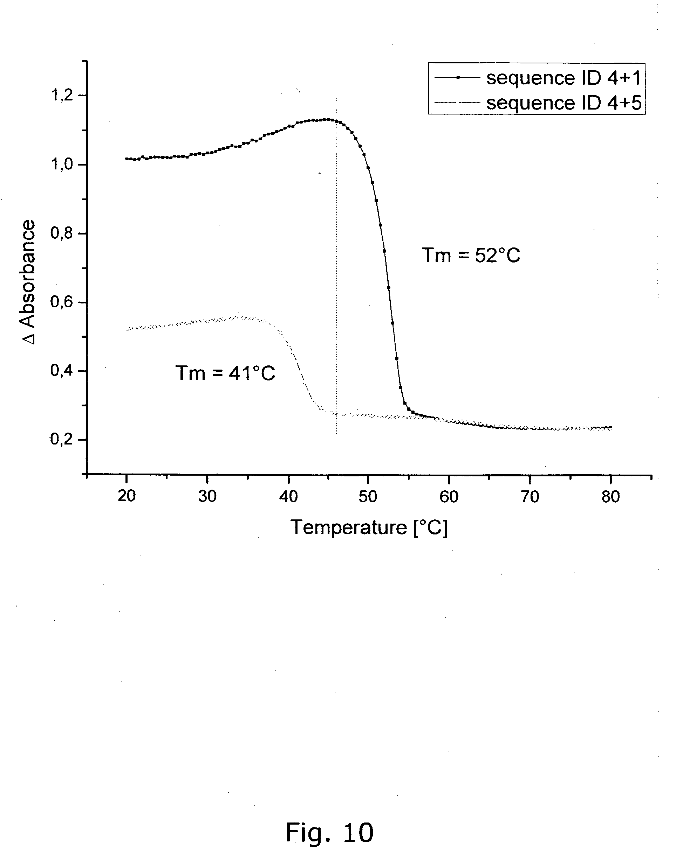

Thermal denaturation curves recorded in a UV experiment at 260 nm wavelength showing hybridisation of sequence ID4 and ID1 compared to the hybridisation of sequence ID4 and ID5 at 0,125 µM DNA concentration of each single stranded DNA sequence (fig. 10).

### Example 5

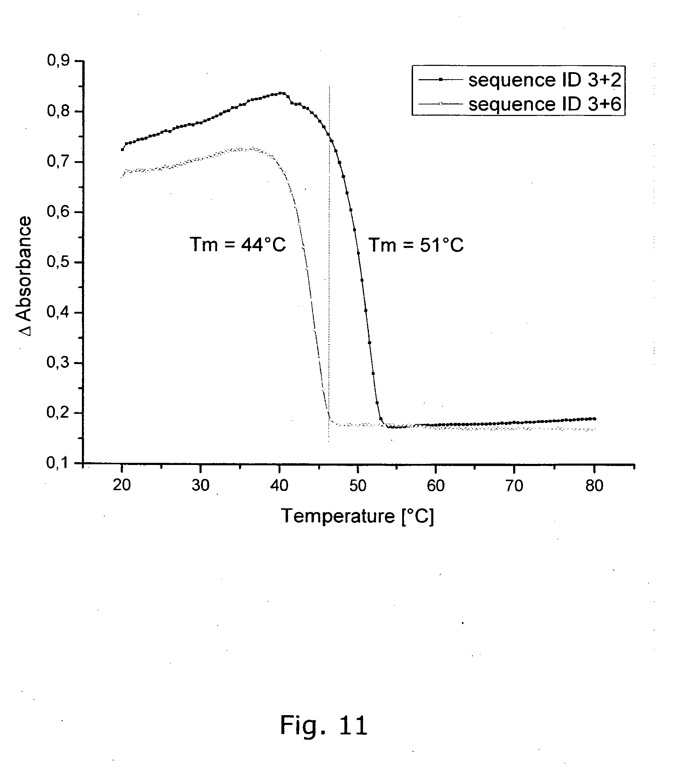

Thermal denaturation curves recorded in a UV experiment at 260 nm wavelength showing hybridisation of sequence ID3 and ID2 compared to the hybridisation of sequence ID3 and ID6 at 0,125 µM DNA concentration of each single stranded DNA sequence (fig. 11)

### Example 6

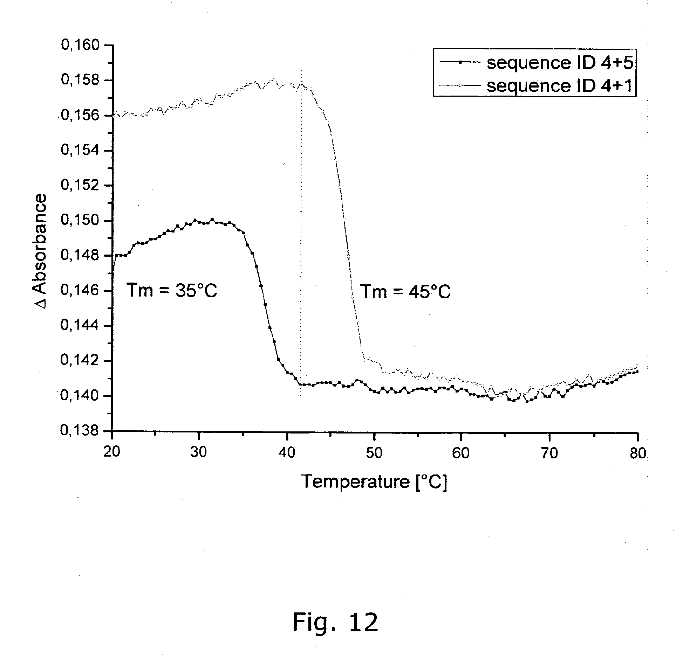

Thermal denaturation curves recorded in a UV experiment at 260 nm wavelength showing hybridisation of sequence ID4 and ID1 compared to the hybridisation of sequence ID4 and ID5 at 12,5 nM DNA concentration of each single stranded DNA sequence (fig. 12).

### Example 7

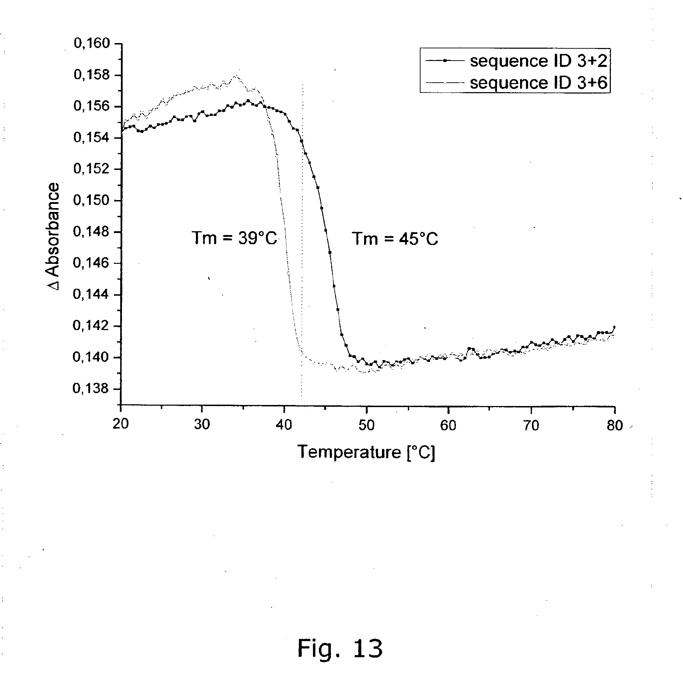

Thermal denaturation curves recorded in a UV experiment at 260 nm wavelength showing hybridisation of sequence ID3 and ID2 compared to the hybridisation of sequence ID3 and ID6 at 0,125 µM DNA concentration of each single stranded DNA sequence (fig. 13).

Example 8. Membrane anchoring moiety is required for assembly of lipid membranes.

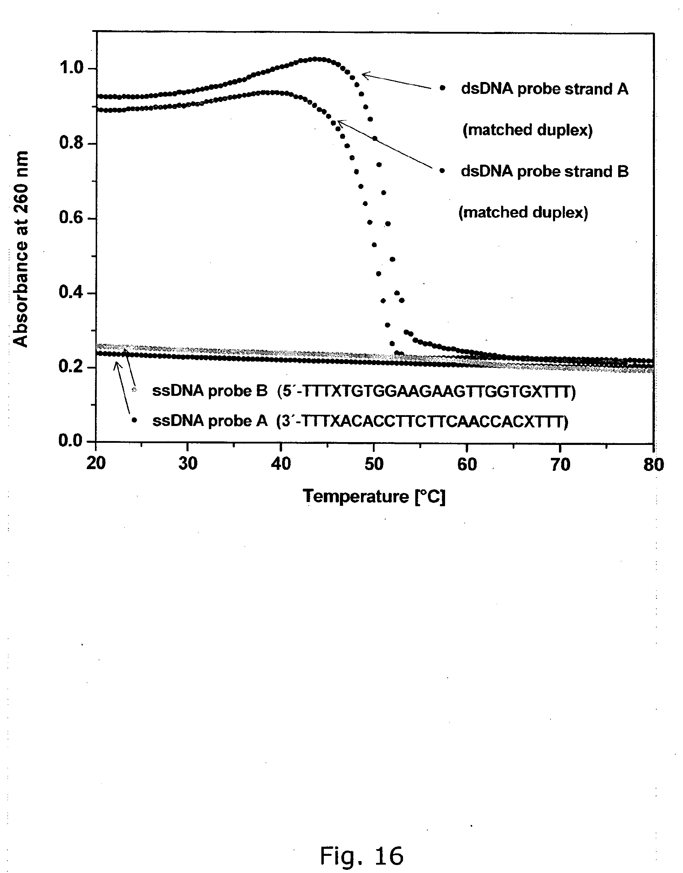

In this experiment, the effect of adding a target oligonucleotide was tested as determined by liposome assembly. As shown in figure 16, lipid assembly only takes when the target oligonucleotide is added.

The experiment was repeated in the presence of serum and yeast cell extract, and neither did affect liposome assembly. Thus, liposome assembly is a robust process that can take place in complex solutions.

### Example 9. Mismatch discrimination studies.

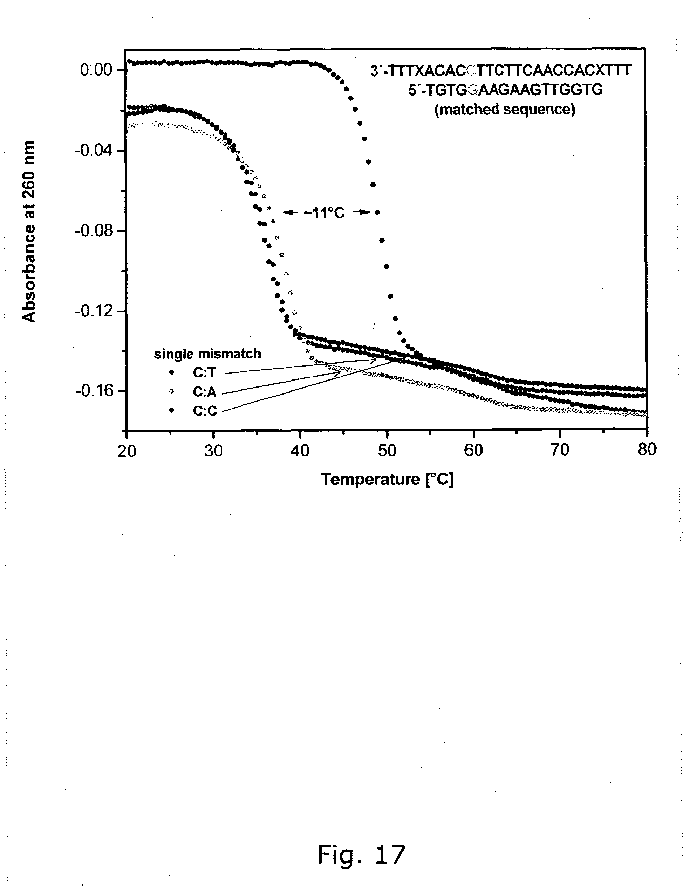

In this experiment, the effect of mismatches was analysed (figure 17). Thus, a perfectly matched target oligonucleotide was compared to three sequences with mismatches. A mismatch decreased the tm value with about 11 °C, seen in the 11 °C shift of the temperature for liposome assembly. The shift in temperature was similar to temperature shift seen for unmodified oligonucleotides.

**Table 1. Mismatch discrimination data**

| Nr. | Duplex | *T*ₘ [°C] | Δ*T*ₘ [° C] | *T*ₘ [° C] | Δ*T*ₘ [° C] |
|---|---|---|---|---|---|
| target | 3'-TTT**X**ACACCTTCTTCAACCAC**X**TTT | with liposomes^{c} | | without liposomes^{d} | |
| 1 | 5'-TGTGGAAGAAGTTGGTG | 48 | - | 47 | - |
| 2 | 5'-TGTG**T**AAGAAGTTGGTG | 36 | -12 | 37 | -10 |
| 3 | 5' -TGTG**A**AAGAAGTTGGTG | 38 | -10 | 38 | -11 |
| 4 | 5' -TGTG**C**AAGAAGTTGGTG | 36 | -12 | 37 | -11 |
| [a] **X** denotes the polyaza crown ether base surrogate. [b] Conditions: *T*m values/° C (Δ*T*m = change in *T*m value calculated relative to the DNA:DNA reference duplex measured as the maximum of the first derivative of the melting curve (*A*₂₆₀ *vs*. temperature) recorded in medium salt buffer (10 mM HEPES, 110 mM Na⁺, pH 7.0). [c] 62 nM concentrations of the two complementary strands in presence of liposomes^{[d]} 1 µM concentrations of the two complementary strands in absence of liposomes. Exp. error: ± 1° C. | | | | | |

### Example 10. Detection of an Anthrax sequence

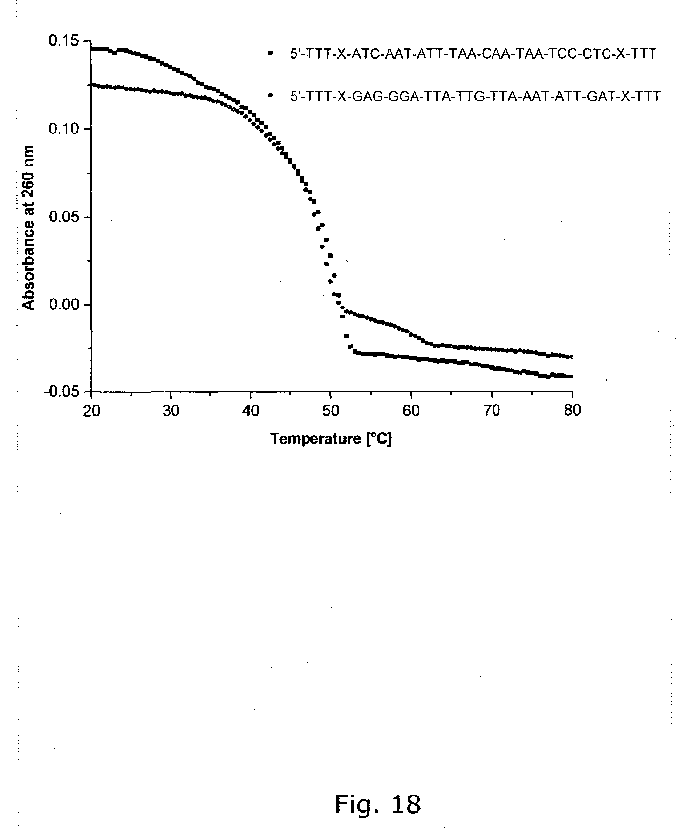

In this experiment, lipid assembly was analysed for a target sequence of Anthrax bacteria. As seen in figure 18, lipid assembly also took place with an Anthrax sequence.

### Example 11. Detection of sequences from Staphlycoccus aureus.

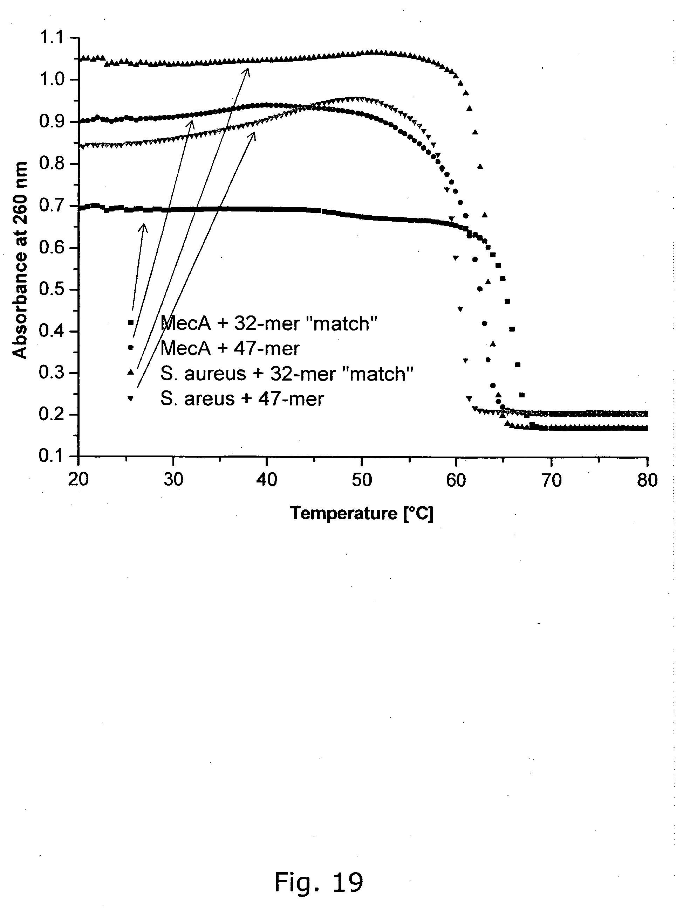

In this experiment, lipid assembly was analysed for a target sequence of Staphlycoccus aureus bacteria. As seen in figure 19 lipid assembly also took place with an Anthrax sequence.

### Example 12. Detection of longer fragments

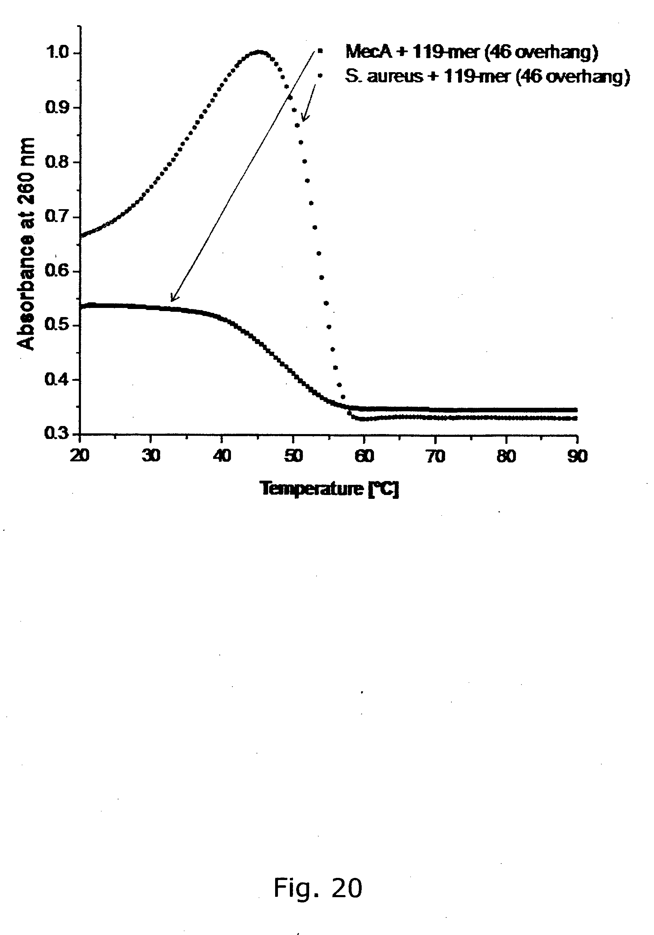

In this experiment, lipid assembly was analysed for a target sequence of S.aureus (figure 20). As an extra feature, the target oligonucleotide was rather long, i.e. 119 nucleotides. Again liposome assembly took place. Thus, the method can also be employed for target fragments that are substantially longer than the amphiphilic oligonucleotide probe.

### Example 13. 3-strand design

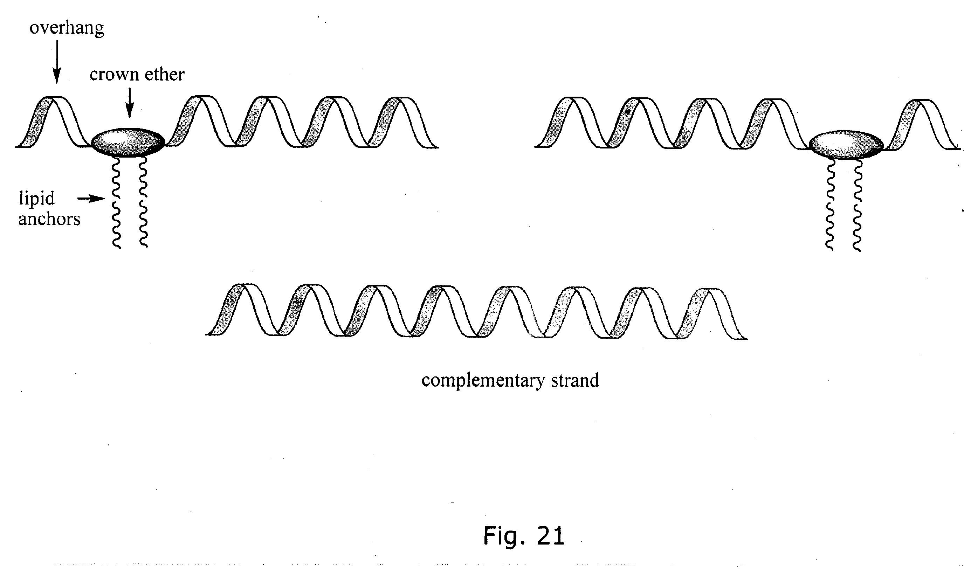

In this experiment, the amphiphilic oligonucleotide was represented by two oligonucleotides, each with complementary to the target oligonucleotide and each with a membrane anchor (figure 20). As shown in figure 21, the use of the two aforementioned oligonucleotides also mediated liposome assembly.

### Example 14. Solid supports

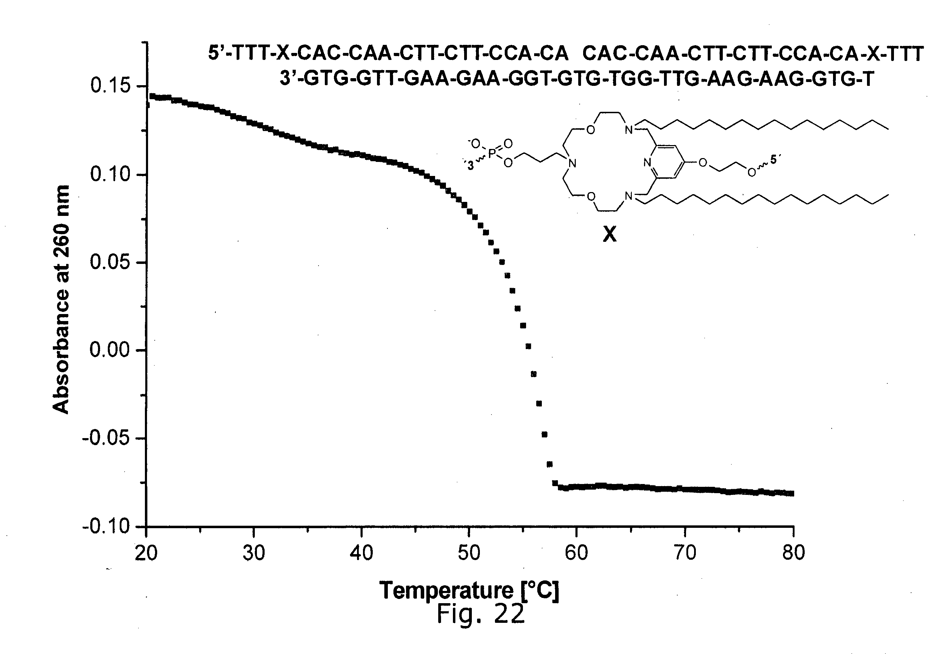

This example demonstrates immobilization of oligonucleotides of the invention to a supported membrane (figure 22). When a target oligonucleotide is added, liposomes where tethered to the surface within a time of 1-2 hours. About 90% assembly occurs within the first 15 minutes.

## Claims

1. A method for quantifying the presence of a particular nucleic acid comprising the steps of:
I. constructing oligonucleotide-lipid architectures comprising
a. providing a multitude of amphiphilic single stranded oligonucleotides, wherein the length of the single stranded amphiphilic oligonucleotides is less than 35 nucleotides, together comprising at least two lipophilic membrane anchors, wherein said membrane anchors each comprises two lipophilic groups;
b. providing a multitude of lipid membranes, wherein said lipid membranes are part of lipid vesicles;
c. incubating the multitude of single stranded amphiphilic oligonucleotides with the multitude of lipid membranes, under conditions allowing insertion of the lipophilic membrane anchors into the lipid membranes, forming a reversible oligonucleotide-lipid membrane architecture, such that the particular membrane anchor may change position from one lipid membrane to another lipid membrane;
II. incubating, under conditions of hybridisation, the multitude of single stranded amphiphilic oligonucleotides and the multitude of lipid membranes with a multitude of nucleic acids that comprise a region of complementarity to the multitude of single strandedamphiphilic oligonucleotides, wherein the region of complementarity has a length of at least 10 nucleotides, creating a targeted nucleic acid hybridization controlled lipid membrane aggregation of the multitude of lipid vesicles;
III. detecting lipid membrane aggregation, by measuring optical density; and
IV. comparing the measured optical density to a standard curve prepared by measurements on oligonucleotide-lipid membrane aggregation using predetermined amounts of a target nucleic acid, thereby quantifying the particular nucleic acid.

2. The method according to claim 1, wherein the membrane anchors are lipophilic moieties comprising an alkyl chain with a length selected from the group consisting of: at least 4 C atoms, at least 5 C-atoms, at least 6 C-atoms, at least 7 C-atoms, at least 8 C-atoms, at least 9 C-atoms, at least 10 C-atoms, at least 11 C-atoms, at least 12 C-atoms, at least 13 C-atoms, at least 14 C-atoms, at least 15 C-atoms, at least 16 C-atoms, at least 17 C-atoms, at least 18 C-atoms, at least 19 C-atoms and at least 20 C-atoms, an alkenyl chain with a length selected from the group consisting of: at least 4 C atoms, at least 5 C-atoms, at least 6 C-atoms, at least 7 C-atoms, at least 8 C-atoms, at least 9 C-atoms, at least 10 C-atoms, at least 11 C-atoms, at least 12 C-atoms, at least 13 C-atoms, at least 14 C-atoms, at least 15 C-atoms, at least 16 C-atoms, at least 17 C-atoms, at least 18 C-atoms, at least 19 C-atoms and at least 20 C-atoms, an alkynyl chain with a length selected from the group consisting of: at least 4 C atoms, at least 5 C-atoms, at least 6 C-atoms, at least 7 C-atoms, at least 8 C-atoms, at least 9 C-atoms, at least 10 C-atoms, at least 11 C-atoms, at least 12 C-atoms, at least 13 C-atoms, at least 14 C-atoms, at least 15 C-atoms, at least 16 C-atoms, at least 17 C-atoms, at least 18 C-atoms, at least 19 C-atoms and at least 20 C-atoms.

3. The method of any of the preceding claims, wherein the membrane anchors are lipophilic moieties comprising one or more aromatic rings.

4. The method of any of the preceding claims, wherein the lipophilic moiety comprises a fatty acid selected from the group consisting of: butanoic acid; hexanoic acid; octanoic acid; decanoic acid; dodecanoic acid; tetradecanoic acid; hexadecanoic acid; 9-hexadecenoic acid; octadecanoic acid; 9-octadecenoic acid; 11-octadecenoic acid; 9,12-octadecadienoic acid; 9,12,15-octadecatrienoic acid; 6,9,12-octadecatrienoic acid; eicosanoic acid; 9-eicosenoic acid; 5,8,11,14-eicosatetraenoic acid; 5,8,11,14,17-eicosapentaenoic acid; docosanoic acid; 13-docosenoic acid; 4,7,10,13,16,19-docosahexaenoic acid and tetracosanoic acid.

5. The method of any of the preceding claims, wherein at least one membrane anchor comprise a polyaza crown ether with two lipophilic substituents.

6. The method of any of the preceding claims, wherein a first lipophilic membrane anchor is located at least 1 nucleotide from the 5'end of the amphiphilic oligonucleotide and wherein a second lipophilic membrane anchor is located at least 1 nucleotides from the 3'end of the amphiphilic oligonucleotide.

7. The method according to any of the preceding claims, wherein the vesicle comprises a detectable marker selected from the group consisting of: a fluorescent group, a quantum dot and a metalnanoparticle.

8. The method of any of the preceding claims, wherein the amphiphilic oligonucleotide comprises monomers selected from the group consisting of DNA, RNA, LNA, PNA, and morpholino.

## Patentansprüche

1. Verfahren zum Quantifizieren der Gegenwart einer bestimmten Nukleinsäure, umfassend folgende Schritte:
I. Konstruieren von Oligonukleotid-Lipid-Architekturen, umfassend
a. Bereitstellen einer Mehrzahl von amphiphilen einsträngigen Oligonukleotiden, wobei die Länge der einsträngigen amphiphilen Oligonukleotide weniger als 35 Nukleotide beträgt, die gemeinsam mindestens zwei lipophile Membrananker umfassen, wobei die Membrananker jeweils zwei lipophile Gruppen umfassen;
b. Bereitstellen einer Mehrzahl von Lipidmembranen, wobei die Lipidmembranen ein Teil von Lipidvesikeln sind;
c. Inkubieren der Mehrzahl von einsträngigen amphiphilen Oligonukleotiden mit der Mehrzahl von Lipidmembranen unter Bedingungen, die das Einsetzen der lipophilen Membrananker in die Lipidmembranen ermöglichen, wobei eine reversible Oligonukleotid-Lipidmembran-Architektur derart ausgebildet wird, dass der bestimmte Membrananker seine Position von einer Lipidmembran zu einer anderen Lipidmembran ändern kann;
II. Inkubieren der Mehrzahl von einsträngigen amphiphilen Oligonukleotiden und der Mehrzahl von Lipidmembranen mit einer Mehrzahl von Nukleinsäuren, die eine zu der Mehrzahl von einsträngigen amphiphilen Oligonukleotiden komplementäre Region umfassen, unter Hybridisierungsbedingungen, wobei die komplementäre Region eine Länge von mindestens 10 Nukleotiden aufweist, wobei ein durch Nukleinsäurehybridisierung gesteuertes Ziel-Lipidmembranaggregat der Mehrzahl von Lipidvesikeln gebildet wird;
III. Nachweis des Lipidmembranaggregats durch Messen der optischen Dichte und
IV. Vergleichen der gemessenen optischen Dichte mit einer Standardkurve, die durch Messungen mit Oligonukleotid-Lipidmembran-Aggregaten unter Verwendung vorbestimmter Mengen einer Zielnukleinsäure erstellt wurde, wodurch die bestimmte Nukleinsäure quantifiziert wird.

2. Verfahren nach Anspruch 1, wobei die Membrananker lipophile Gruppierungen mit einer Alkylkette mit einer Länge ausgewählt aus der Gruppe, bestehend aus: mindestens 4 C-Atomen, mindestens 5 C-Atomen, mindestens 6 C-Atomen, mindestens 7 C-Atomen, mindestens 8 C-Atomen, mindestens 9 C-Atomen, mindestens 10 C-Atomen, mindestens 11 C-Atomen, mindestens 12 C-Atomen, mindestens 13 C-Atomen, mindestens 14 C-Atomen, mindestens 15 C-Atomen, mindestens 16 C-Atomen, mindestens 17 C-Atomen, mindestens 18 C-Atomen, mindestens 19 C-Atomen and mindestens 20 C-Atomen, einer Alkenylkette mit einer Länge ausgewählt aus der Gruppe, bestehend aus: mindestens 4 C-Atomen, mindestens 5 C-Atomen, mindestens 6 C-Atomen, mindestens 7 C-Atomen, mindestens 8 C-Atomen, mindestens 9 C-Atomen, mindestens 10 C-Atomen, mindestens 11 C-Atomen, mindestens 12 C-Atomen, mindestens 13 C-Atomen, mindestens 14 C-Atomen, mindestens 15 C-Atomen, mindestens 16 C-Atomen, mindestens 17 C-Atomen, mindestens 18 C-Atomen, mindestens 19 C-Atomen and mindestens 20 C-Atomen, einer Alkinylkette mit einer Länge ausgewählt aus der Gruppe, bestehend aus: mindestens 4 C-Atomen, mindestens 5 C-Atomen, mindestens 6 C-Atomen, mindestens 7 C-Atomen, mindestens 8 C-Atomen, mindestens 9 C-Atomen, mindestens 10 C-Atomen, mindestens 11 C-Atomen, mindestens 12 C-Atomen, mindestens 13 C-Atomen, mindestens 14 C-Atomen, mindestens 15 C-Atomen, mindestens 16 C-Atomen, mindestens 17 C-Atomen, mindestens 18 C-Atomen, mindestens 19 C-Atomen and mindestens 20 C-Atomen, sind.

3. Verfahren nach einem der vorhergehenden Ansprüche, wobei die Membrananker lipophile Gruppierungen mit einem oder mehreren aromatischen Ringen sind.

4. Verfahren nach einem der vorhergehenden Ansprüche, wobei die lipophile Gruppierung eine Fettsäure umfasst, ausgewählt aus der Gruppe, bestehend aus: Butansäure; Hexansäure; Octansäure; Decansäure; Dodecansäure; Tetradecansäure; Hexadecansäure; 9-Hexadecensäure; Octadecansäure; 9-Octadecensäure; 11-Octadecensäure; 9,12-Octadecadiensäure; 9,12,15-Octadecatriensäure; 6,9,12-Octadecatriensäure; Eicosansäure; 9-Eicosensäure; 5,8,11,14-Eicosatetraensäure; 5,8,11,14,17-Eicosapentaensäure; Docosansäure; 13-Docosensäure; 4,7,10,13,16,19-Docosahexaensäure und Tetracosansäure.

5. Verfahren nach einem der vorhergehenden Ansprüche, wobei mindestens ein Membrananker einen Polyazakronenether mit zwei lipophilen Substituenten umfasst.

6. Verfahren nach einem der vorhergehenden Ansprüche, wobei sich ein erster lipophiler Membrananker mindestens 1 Nukleotid vom 5'-Ende des amphiphilen Oligonukleotids befindet und wobei ein sich ein zweiter lipophiler Membrananker mindestens 1 Nukleotid vom 3'-Ende des amphiphilen Oligonukleotids befindet.

7. Verfahren nach einem der vorhergehenden Ansprüche, wobei das Vesikel einen erfassbaren Marker umfasst, ausgewählt aus der Gruppe, bestehend aus: einer fluoreszierenden Gruppe, einem Quantenpunkt und einem Metallnanopartikel.

8. Verfahren nach einem der vorhergehenden Ansprüche, wobei das amphiphile Oligonukleotid Monomere umfasst, ausgewählt aus der Gruppe, bestehend aus DNA, RNA, LNA, PNA und Morpholino.

## Revendications

1. Procédé destiné à quantifier la présence d'un acide nucléique particulier comprenant les étapes suivantes :
I. construction d'architectures lipides-oligonucléotides comprenant
a. l'apport d'une multitude d'oligonucléotides amphiphiles simple brin, où la longueur des oligonucléotides amphiphiles simple brin est inférieure à 35 nucléotides, comprenant en tout au moins deux ancrages membranaires lipophiles, où lesdits ancrages membranaires comprennent chacun deux groupes lipophiles ;
b. l'apport d'une multitude de membranes lipidiques, où lesdites membranes lipidiques font partie de vésicules lipidiques ;
c. l'incubation de la multitude d'oligonucléotides amphiphiles simple brin avec la multitude de membranes lipidiques, dans des conditions permettant l'insertion des ancrages membranaires lipophiles dans les membranes lipidiques, formant une architecture de membranes lipidiques-oligonucléotides réversible, de manière à ce que l'ancrage membranaire concerné puisse changer de position d'une membrane lipidique vers une autre membrane lipidique ;
II. incubation, dans des conditions d'hybridation, de la multitude d'oligonucléotides amphiphiles simple brin et de la multitude de membranes lipidiques avec une multitude d'acides nucléiques qui comprennent une région de complémentarité avec la multitude d'oligonucléotides amphiphiles simple brin, où la région de complémentarité présente une longueur d'au moins 10 nucléotides, créant une agrégation cible de membranes lipidiques de la multitude de vésicules lipidiques contrôlée par hybridation à un acide nucléique ;
III. détection de l'agrégation de membranes lipidiques, par mesure de la densité optique ; et
IV. comparaison de la densité optique mesurée à une courbe standard préparée en réalisant des mesures sur une agrégation de membranes lipidiques-oligonucléotides à l'aide de quantités prédéterminées d'un acide nucléique cible, en quantifiant ainsi l'acide nucléique concerné.

2. Le procédé selon la revendication 1, où les ancrages membranaires sont des fractions lipophiles comprenant une chaîne alkyle de longueur choisie dans le groupe consistant en : au moins 4 atomes de C, au moins 5 atomes de C, au moins 6 atomes de C, au moins 7 atomes de C, au moins 8 atomes de C, au moins 9 atomes de C, au moins 10 atomes de C, au moins 11 atomes de C, au moins 12 atomes de C, au moins 13 atomes de C, au moins 14 atomes de C, au moins 15 atomes de C, au moins 16 atomes de C, au moins 17 atomes de C, au moins 18 atomes de C, au moins 19 atomes de C et au moins 20 atomes de C, une chaîne alcényle de longueur choisie dans le groupe consistant en : au moins 4 atomes de C, au moins 5 atomes de C, au moins 6 atomes de C, au moins 7 atomes de C, au moins 8 atomes de C, au moins 9 atomes de C, au moins 10 atomes de C, au moins 11 atomes de C, au moins 12 atomes de C, au moins 13 atomes de C, au moins 14 atomes de C, au moins 15 atomes de C, au moins 16 atomes de C, au moins 17 atomes de C, au moins 18 atomes de C, au moins 19 atomes de C et au moins 20 atomes de C, une chaîne alcynyle de longueur choisie dans le groupe consistant en : au moins 4 atomes de C, au moins 5 atomes de C, au moins 6 atomes de C, au moins 7 atomes de C, au moins 8 atomes de C, au moins 9 atomes de C, au moins 10 atomes de C, au moins 11 atomes de C, au moins 12 atomes de C, au moins 13 atomes de C, au moins 14 atomes de C, au moins 15 atomes de C, au moins 16 atomes de C, au moins 17 atomes de C, au moins 18 atomes de C, au moins 19 atomes de C et au moins 20 atomes de C.

3. Le procédé de l'une quelconque des revendications précédentes, où les ancrages membranaires sont des fractions lipophiles comprenant un ou plusieurs cycles aromatiques.

4. Le procédé de l'une quelconque des revendications précédentes, où la fraction lipophile comprend un acide gras choisi dans le groupe consistant en : acide butanoïque ; acide hexanoïque ; acide octanoïque ; acide décanoïque ; acide dodécanoïque ; acide tétradécanoïque ; acide hexadécanoïque ; acide 9-hexadécénoïque ; acide octadécanoïque ; acide 9-octadécénoïque ; acide 11-octadécénoïque ; acide 9,12-octadécadiénoïque ; acide 9,12,15-octadécatriénoïque ; acide 6,9,12-octadécatriénoïque ; acide eicosanoïque ; acide 9-eicosénoïque ; acide 5,8,11,14-eicosatétraénoïque ; acide 5,8,11,14,17-eicosapentaénoïque ; acide docosanoïque ; acide 13-docosénoïque ; acide 4,7,10,13,16,19-docosahexaénoïque et acide tétracosanoïque.

5. Le procédé de l'une quelconque des revendications précédentes, où au moins un ancrage membranaire comprend un éther-couronne polyazoté avec deux substituants lipophiles.

6. Le procédé de l'une quelconque des revendications précédentes, où un premier ancrage membranaire lipophile est situé à au moins 1 nucléotide de l'extrémité 5' de l'oligonucléotide amphiphile et où un deuxième ancrage membranaire lipophile est situé à au moins 1 nucléotide de l'extrémité 3' de l'oligonucléotide amphiphile.

7. Le procédé selon l'une quelconque des revendications précédentes, où la vésicule comprend un marqueur détectable choisi dans le groupe consistant en : un groupe fluorescent, un point quantique et une nanoparticule métallique.

8. Le procédé de l'une quelconque des revendications précédentes, où l'oligonucléotide amphiphile comprend des monomères choisis dans le groupe consistant en ADN, ARN, LNA, PNA et morpholino.


### SEQUENCE LISTING

<110> Syddansk Universitet, Odense
Vogel, Stefan
Cohen Simonsen, Adam
<120> DNA Controlled assembly of lipid membranes
<130> 41752PC01
<160> 11
<170> PatentIn version 3.5
<210> 1
   <211> 17
   <212> DNA
   <213> Artificial Sequence
<220>
   <223> Synthetic oligonucleotide
<400> 1
   tgtggaagaa gttggtg 17
<210> 2
   <211> 17
   <212> DNA
   <213> Artificial Sequence
<220>
   <223> Synthetic oligonucleotide
<400> 2
   caccaacttc ttccaca 17
<210> 3
   <211> 17
   <212> DNA
   <213> Artificial Sequence
<220>
   <223> Synthetic oligonucleotide
<400> 3
   tgtgtaagaa gttggtg 17
<210> 4
   <211> 17
   <212> DNA
   <213> Artificial Sequence
<220>
   <223> Synthetic oligonucleotide
<400> 4
   cacctacttc ttccaca 17
<210> 5
   <211> 23
   <212> DNA
   <213> Artificial Sequence
<220>
   <223> Synthetic oligonucleotide
<400> 5
   ttttgtggaa gaagttggtg ttt 23
<210> 6
   <211> 23
   <212> DNA
   <213> Artificial Sequence
<220>
   <223> Synthetic oligonucleotide
<400> 6
   tttcaccaac ttcttccaca ttt 23
<210> 7
   <211> 30
   <212> DNA
   <213> Artificial Sequence
<220>
   <223> Synthetic oligonucleotide
<400> 7
   tttatcaata tttaacaata atccctcttt 30
<210> 8
   <211> 30
   <212> DNA
   <213> Artificial Sequence
<220>
   <223> Synthetic oligonucleotide
<400> 8
   tttgagggat tattgttaaa tattgatttt 30
<210> 9
   <211> 20
   <212> DNA
   <213> Artificial Sequence
<220>
   <223> Synthetic oligonucleotide
<400> 9
   tttcaccaac ttcttccaca 20
<210> 10
   <211> 20
   <212> DNA
   <213> Artificial Sequence
<220>
   <223> Synthetic oligonucleotide
<400> 10
   caccaacttc ttccacattt 20
<210> 11
   <211> 34
   <212> DNA
   <213> Artificial Sequence
<220>
   <223> Synthetic oligonucleotide
<400> 11
   tgtggaagaa gttggtgtgt ggaagaagtt ggtg 34